# EUROPEAN PATENT APPLICATION

(11) **EP 4 851 182 A1**
(43) Date of publication of application: **09.12.2026**
(21) Application number: 25751509.8
(22) Date of filing: 05.02.2025
(51) Int. Cl.: C07K 14/155, C07K 14/005, A61P 31/12, A61K 39/21, A61P 31/18, A61P 31/14, C12N 15/49

(54) **ORAL LONG-ACTING POLYPEPTIDE HIV INACTIVATOR**

(30) Priority: 05.02.2024 WO PCT/CN2024/076010
(71) Applicant: Academy of Military Medical Sciences, Beijing 100850 (CN)
(72) Inventor: WANG, Chao, Beijing 100850 (CN); ZHANG, Wenpeng, Beijing 100850 (CN); ZHUANG, Xiaomei, Beijing 100850 (CN); LI, Qing, Beijing 100850 (CN); TU, Jiahuang, Beijing 100850 (CN)
(74) Representative: Prüfer & Partner mbB Patentanwälte · Rechtsanwälte
(86) International application number: PCT/CN2025/075786
(87) International publication number: WO 2025/167910

(57) **Abstract**

Provided are a novel long-acting HIV inactivating or inhibiting polypeptide that can be taken orally or has oral potential, a stereoisomer, derivative, pharmaceutically acceptable salt, mixture, or functionally equivalent variant of the polypeptide, and a pharmaceutical composition, drug conjugate, drug complex, or fusion protein containing the above. Further, provided are a pharmaceutical use and a synthesis method of the polypeptide, the stereoisomer, derivative, pharmaceutically acceptable salt, mixture, or functionally equivalent variant of the polypeptide and the pharmaceutical composition, drug conjugate, drug complex, or fusion protein for treating or preventing diseases caused by HIV infection.

## Description

### CROSS-REFERENCES

The present application claims priority to PCT application No. PCT/CN2024/076010, filed on February 5, 2024. All contents of prior applications are considered to be the disclosure of the present application and are incorporated herein in their entirety.

### SEQUENCE LISTING REFERENCE

The sequence listing named DCF231808WO-Sequence Listing.xml, created on January 18, 2024, with 17,295 bytes in size is hereby incorporated in its entirety by reference.

### TECHNICAL FIELD

The present disclosure relates to the biomedical field, specifically to polypeptide HIV inactivators and inhibitors.

### BACKGROUND

Acquired immunodeficiency syndrome is a major infectious disease caused by infection with the human immunodeficiency virus (HIV). Since the first case of acquired immunodeficiency syndrome patient was identified, the global death toll has exceeded 40 million. There are still 39 million people living with acquired immunodeficiency syndrome worldwide, and as many as 1.3 million new infections were reported in 2023. Acquired immunodeficiency syndrome has an extremely high mortality rate, remains incurable, and there is currently no effective vaccine available. Drug therapy remains the only effective method at present. Based on studies of the viral life cycle, five classes of anti-HIV drugs or HIV therapeutic agents have been developed: entry inhibitors, reverse transcriptase inhibitors, integrase inhibitors, protease inhibitors, and capsid inhibitors. Existing anti-HIV clinical drugs or HIV therapeutic agents act only during the process of viral infection of host cells or after the virus has entered the host cells. Therefore, the drugs or agents function merely as passive defenses and are unable to actively attack the virus in the absence of host cells, directly inactivate free viral particles, or render the virus non-infectious. In addition, due to poor penetration of drugs into specific tissues or organs, such as the central nervous system, the virus can continue to replicate at a low level in these tissues or organs. These tissues or anatomical sites that are difficult for drugs to reach constitute HIV reservoirs. The existence of HIV reservoirs is a major factor contributing to persistent HIV infection and represents one of the greatest obstacle to the complete eradication of HIV and the achievement of a cure for acquired immunodeficiency syndrome.

The viral envelope glycoprotein (Env) plays an important role in the process of HIV invading host cells. HIV Env consists of a surface subunit gp120 and a transmembrane subunit gp41. The transmembrane subunit gp41 comprises multiple functional regions in its sequence structure, including the N-terminal fusion peptide (FP) region, the N-terminal heptad repeat (NHR) region, the C-terminal heptad repeat (CHR) region, the tryptophan-rich region, the transmembrane (TM) region, and the cytoplasmic tail (CT) region, etc. Recently, researchers have screened a 15-amino-acid polypeptide, F9170 (amino acid sequence: GWEALKYLWNLLQYW, SEQ ID NO: 5), from a polypeptide library. The polypeptide not only exhibits effective HIV inhibitory activity, but also has the ability to inactivate HIV viral particles. Sequence analysis showed that the polypeptide is derived from the lentiviral lytic peptide-3 (LLP-3) region of the gp41 CT domain. The core mechanism of F9170 is likely associated with the interaction with the lentiviral lytic peptide-1 (LLP-1) region within the gp41 CT domain, disrupting the integrity of the viral membrane and causing the virus to lose infectivity. However, the F9170 polypeptide has druggability defects such as a short in vivo half-life and the need for frequent injection administration, which severely restrict its further clinical application. Accordingly, there is an urgent need to develop highly effective and safe HIV inactivators and inhibitors with ease of administration and high patient compliance.

### SUMMARY

The present disclosure provides a polypeptide that can serve as a novel HIV inactivator or inhibitor and has one or more of the following advantages: simple synthesis, low synthesis cost, oral administration capability or oral administration potential, highly effective HIV inhibitory activity, highly effective lysis of HIV viral particles, highly effective inactivation of free HIV viral particles, long-acting effect, effective entry into an HIV anatomical reservoir, and good biosafety. Compared with F9170, the polypeptide provided in the present disclosure has the advantages of significantly improved in vivo half-life, oral administration capability or oral administration potential, and/or effective entry into an HIV anatomical reservoir.

In one aspect, the present disclosure provides a polypeptide or a stereoisomer, derivative, pharmaceutically acceptable salt, mixture, or functionally equivalent variant thereof, the polypeptide comprising an amino acid sequence represented by any one of the following general formulas (I)-(IV), or an amino acid sequence represented by any one of the general formulas (I)-(IV) having 1, 2, 3, 4, or 5 conservative substitutions:
X₁-X₂-X₃-X₄-X₅-X₆-X₇-X₈-X₉-X₁₀-X₁₁-X₁₂-X₁₃-X₁₄-W (I),
X₂-W-E-A-X₆-K-X₉-L-W-N-X₁₃-L-Q-Y-W (II),
G-X₂-E-A-L-X₆-Y-L-W-N-L-L-Q-Y-W (III),
G-W-E-A-L-K-Y-L-X₉-N-L-L-X₁₃-Y-W (IV),
wherein X₁ represents any amino acid, preferably G or W, or an amino acid residue having similar properties thereto; X₂, X₆, X₉, and X₁₃ are independently non-natural amino acids, wherein (X₂) and (X₆), and (X₉) and (X₁₃) are bonded to each other via a covalent bond linkage, respectively; X₃ represents any amino acid, preferably E or R, or an amino acid residue having similar properties thereto; X₄ represents any amino acid, preferably A or W, or an amino acid residue having similar properties thereto; X₅ represents any amino acid, preferably L or W, or an amino acid residue having similar properties thereto; X₇ represents any amino acid, preferably Y or R, or an amino acid residue having similar properties thereto; X₈ represents any amino acid, preferably L or W, or an amino acid residue having similar properties thereto; X₁₀ represents any amino acid, preferably N or R, or an amino acid residue having similar properties thereto; X₁₁ represents any amino acid, preferably L, W, or R, or an amino acid residue having similar properties thereto; X₁₂ represents any amino acid, preferably L or W, or an amino acid residue having similar properties thereto; X₁₄ represents any amino acid, preferably Y or R, or an amino acid residue having similar properties thereto; and the symbol "-" represents a peptide bond between amino acid residues.

In some preferred embodiments, the polypeptide provided in the present disclosure has at least 40%, 50%, 60%, 70%, 71%, 72%, 73%, 80%, or 90% sequence identity to F9170 (SEQ ID NO: 5).

In another aspect, the present disclosure provides a fusion protein comprising the polypeptide disclosed herein, or the stereoisomer, derivative, pharmaceutically acceptable salt, mixture, or functionally equivalent variant of the polypeptide. In an embodiment, the fusion protein may optionally comprise one or more additional proteins for treating or preventing HIV infection, such as an antibody against an intracellular domain of an HIV envelope protein, preferably an antibody against LLP1-GQ, such as the LLP1-GQ antibody set forth in SEQ ID NO: 4 (GACRAIRHIPRRIRQ).

In another aspect, the present disclosure provides a pharmaceutical composition comprising at least the polypeptide disclosed herein, or the stereoisomer, derivative, pharmaceutically acceptable salt, mixture, or functionally equivalent variant of the polypeptide, and a pharmaceutically acceptable carrier or excipient. In an embodiment, the pharmaceutical composition may optionally further comprise one or more additional HIV therapeutic agents or HIV latency-reversing agents.

In another aspect, the present disclosure provides a drug conjugate comprising at least the polypeptide disclosed herein, or the stereoisomer, derivative, pharmaceutically acceptable salt, mixture, or functionally equivalent variant of the polypeptide. In an embodiment, the drug conjugate may optionally comprise a carrier protein, such as serum albumin, immunoglobulin, ferritin, transferrin, α-2-macroglobulin, thyroxine-binding protein, or steroid-binding protein. In an embodiment, the drug conjugate may optionally comprise one or more additional HIV therapeutic agents or HIV latency-reversing agents.

In one aspect, the present disclosure provides a drug complex comprising at least the polypeptide disclosed herein, or the stereoisomer, derivative, pharmaceutically acceptable salt, mixture, or functionally equivalent variant of the polypeptide. In an embodiment, the drug complex may optionally comprise a carrier protein, such as serum albumin, immunoglobulin, ferritin, transferrin, α-2-macroglobulin, thyroxine-binding protein, or steroid-binding protein. In an embodiment, the drug complex may optionally comprise one or more additional HIV therapeutic agents or HIV latency-reversing agents.

In an embodiment, the pharmaceutical composition, drug conjugate, drug complex, or fusion protein disclosed herein can be independently formulated into dosage forms comprising tablets, pills, powders, suppositories, solutions, suspensions, emulsions, granules, tinctures, capsules, transdermal preparations, aerosols, effervescent tablets, drops, and lyophilized powders.

In one aspect, the present disclosure provides the use of the polypeptide disclosed herein, or the stereoisomer, derivative, pharmaceutically acceptable salt, mixture, or functionally equivalent variant of the polypeptide, in the preparation of a medicament for treating or preventing diseases caused by HIV infection. In an embodiment, the dosage forms of the medicament comprise tablets, pills, powders, suppositories, solutions, suspensions, emulsions, granules, tinctures, capsules, transdermal preparations, aerosols, effervescent tablets, drops, and lyophilized powders.

In another aspect, the present disclosure provides the use of the polypeptide disclosed herein, or the stereoisomer, derivative, pharmaceutically acceptable salt, mixture, or functionally equivalent variant of the polypeptide, as a medicament for treating or preventing diseases caused by HIV infection.

In another aspect, the present disclosure provides a method for treating or preventing diseases caused by HIV infection, comprising administering to a subject in need thereof a therapeutically effective amount of the polypeptide disclosed herein, or the stereoisomer, derivative, pharmaceutically acceptable salt, mixture, or functionally equivalent variant of the polypeptide, or the pharmaceutical composition, drug conjugate, drug complex, or fusion protein disclosed herein. In an embodiment, the polypeptide disclosed herein, or the stereoisomer, derivative, pharmaceutically acceptable salt, mixture, or functionally equivalent variant of the polypeptide, the pharmaceutical composition, drug conjugate, drug complex, or fusion protein disclosed herein, is administered in the following ways: injection administration, cavity administration, respiratory tract administration, mucosal administration, or surface administration, preferably injection administration.

In another aspect, the present disclosure provides methods for in vitro use for any one or more of the following (a)-(e): (a) inhibiting HIV infection and/or replication; (b) inactivating free HIV viral particles; (c) preparing a formulation having in vitro HIV infection-inhibitory activity; (d) eliminating viruses in an HIV reservoir or reducing the number of viruses in the HIV reservoir; and (e) lysing HIV viral particles.

The method comprises using the polypeptide disclosed herein, or the stereoisomer, derivative, pharmaceutically acceptable salt, mixture, or functionally equivalent variant of the polypeptide.

In one aspect, the present disclosure provides the in vitro use of the polypeptide disclosed herein, or the stereoisomer, derivative, pharmaceutically acceptable salt, mixture, or functionally equivalent variant of the polypeptide, for any one or more of the following (a)-(e): (a) inhibiting HIV infection and/or replication; (b) inactivating free HIV viral particles; (c) preparing a formulation having in vitro HIV infection-inhibitory activity; (d) eliminating viruses in an HIV reservoir or reducing the number of viruses in the HIV reservoir; and (e) lysing HIV viral particles.

In one aspect, the present disclosure provides a method for producing the polypeptide disclosed herein, or the stereoisomer, derivative, pharmaceutically acceptable salt, mixture, or functionally equivalent variant of the polypeptide, or the fusion protein disclosed herein, comprising chemically synthesizing the polypeptide, or the stereoisomer, derivative, pharmaceutically acceptable salt, mixture, or functionally equivalent variant of the polypeptide, or the fusion protein. In an embodiment, the chemical method may be a solid-phase (e.g., Fmoc solid-phase synthesis) or a liquid-phase synthesis. In an embodiment, the method used herein for producing the polypeptide disclosed herein, or the stereoisomer, derivative, pharmaceutically acceptable salt, mixture, or functionally equivalent variant of the polypeptide, or the fusion protein disclosed herein, are methods commonly used in the art.

The polypeptide provided in the present disclosure has at least one of the following significant advantages: (1) it can directly kill free viruses; (2) it has only 15 amino acids and is significantly shorter than other anti-HIV polypeptides, such as T20 (YTSLIHSLIEESQNQQEKNEQELLELDKWASLWNWF (SEQ ID NO: 6)) and T2635 (TTWEAWDRAIAEYAARIEALIRAAQEQQEKNEAALREL (SEQ ID NO: 7)). Hence, the polypeptide of the present invention is simple to synthesize and low in cost; (3) it has good biosafety; (4) compared with F9170 (amino acid sequence: GWEALKYLWNLLQYW, SEQ ID NO: 5), the polypeptide provided in the present disclosure maintains the inhibition of HIV replication and/or inactivation of free HIV viral particles, while significantly prolonging the half-life, which can significantly reduce the number of administrations and improve patient compliance; (5) the polypeptide provided in the present disclosure is an orally absorbable polypeptide or has oral administration potential, which has significant advantages over other similar drugs and is expected to become the first orally absorbable peptide anti-HIV drug; (6) compared with F9170, the polypeptide provided in the present disclosure can effectively enter the HIV anatomical reservoir.

The polypeptide provided in the present disclosure will contribute to the prevention and treatment of AIDS, as well as the elimination or reduction of viruses in latent reservoirs, and accelerate the application of novel anti-HIV drugs and the development of new combined therapy strategies.

### BRIEF DESCRIPTION OF DRAWINGS

The following is a brief description of the drawings, which are used to illustrate the exemplary embodiments disclosed herein and are not intended to limit those embodiments.
FIG. 1 is a schematic diagram of synthesis constructed using a dual stapling strategy in Example 1.
FIG. 2 is a mass spectrum of polypeptide D26 in Example 1.
FIG. 3 is a mass spectrum of polypeptide D1 in Example 1.
FIG. 4 is a mass spectrum of polypeptide F2 in Example 1.
FIG. 5 is a mass spectrum of polypeptide F6 in Example 1.
FIG. 6 is a broken line graph of blood concentrations at different time points after intravenous injection of D26, D1, F2, F6 or F9170 in Example 4. Solid circles represent D26, solid squares represent D1, solid equilateral triangles represent F2, solid inverted triangles represent F6, and solid rhombuses represent F9170.
FIG. 7 is a bar chart showing the area under curve in mouse tissues and organs after intravenous injection of D26 or F9170 in Example 5. Mosaic represents D26, and solid cores represents F9170.
FIG. 8 is a blood concentration-time curve of 50 mg/kg D26 administered by gavage in Example 6. The blood concentration of F9170 is below the limit of detection, where the dashed line represents the lower limit of quantitation.
FIG. 9A is a curve graph showing the change in body weight over time for male and female mice in Example 7 after injection of PBS, 20 mg/kg D26, and 100 mg/kg D26. Solid circles represent the PBS group, solid squares represent the 20 mg/kg D26 group, and solid triangles represent the 100 mg/kg D26 group.
FIG. 9B shows the serum alanine aminotransferase levels of mice in Example 7 at different time points after injection with PBS, 20 mg/kg D26, and 100 mg/kg D26. Solid circles represent the PBS group, solid squares represent the 20 mg/kg D26 group, and solid triangles represent the 100 mg/kg D26 group.
FIG. 9C shows the serum creatinine levels of mice in Example 7 at different time points after injection of PBS, 20 mg/kg D26, and 100 mg/kg D26. Solid circles represent the PBS group, solid squares represent the 20 mg/kg D26 group, and solid triangles represent the 100 mg/kg D26 group.
FIG. 10 is a mass spectrum of polypeptide D26W1 in Example 1.
FIG. 11 is a mass spectrum of polypeptide D26W4 in Example 1.
FIG. 12 is a mass spectrum of polypeptide D26W5 in Example 1.
FIG. 13 is a mass spectrum of polypeptide D26W8 in Example 1.
FIG. 14 is a mass spectrum of polypeptide D26W11 in Example 1.
FIG. 15 is a mass spectrum of polypeptide D26W12 in Example 1.
FIG. 16 is a mass spectrum of polypeptide D26R3 in Example 1.
FIG. 17 is a mass spectrum of polypeptide D26R7 in Example 1.
FIG. 18 is a mass spectrum of polypeptide D26R10 in Example 1.
FIG. 19 is a mass spectrum of polypeptide D26R11 in Example 1.
FIG. 20 is a mass spectrum of polypeptide D26R14 in Example 1.
FIG. 21 is a mass spectrum of polypeptide D26-2R in Example 1.
FIG. 22 is a mass spectrum of polypeptide D26-3R in Example 1.
FIG. 23 is a mass spectrum of polypeptide D26-4R in Example 1.
FIG. 24 is a mass spectrum of polypeptide D26-5R in Example 1.

### DETAILED DESCRIPTION

Unless otherwise specified, all figures used in the description and claims to indicate content, concentration, proportion, weight, particle size, percentage, technical effect, etc., shall in any event be understood to be modified by the terms "about" or "approximately". Accordingly, unless otherwise indicated, the numerical parameters listed in the following description and appended claims are approximate values.

Unless otherwise specified, all terms used herein have the same meaning as commonly understood by those skilled in the art. For those skilled in the art, they may vary depending on the desired nature and effect sought to be obtained through the present application, and each numerical parameter shall be interpreted according to the number of significant figures and conventional rounding methods or in a manner understood by those skilled in the art. Generally, the names used herein and the experimental procedures described herein for organic chemistry, medicinal chemistry, and biology are well known and commonly employed in the art. Unless otherwise defined, all technical and scientific terms used herein generally have the same meaning as commonly understood by one of ordinary skill in the art to which the present application belongs. If multiple definitions exist for a term used herein, the definitions set forth in this section shall prevail unless otherwise specified.

### Definitions

When used herein, the expression "A and/or B" comprises three cases: (1) A; (2) B; and (3) A and B. The expression "A, B, and/or C" comprises seven cases: (1) A; (2) B; (3) C; (4) A and B; (5) A and C; (6) B and C; and (7) A, B, and C. The meaning of similar expressions is analogous.

When used herein, the term "independently" means that multiple events do not affect each other. For example, "X and Y are independently selected from any one of a, b, c, d, e, f, and g" means that X can be any one of a, b, c, d, e, f, and g, and Y can also be any one of a, b, c, d, e, f, and g. The selected X and the selected Y can be the same or different, and they do not interfere with each other.

As used herein, the terms "include" and "comprise" mean that, in addition to the elements listed, other elements are not excluded.

As used herein, the term "identity" refers to the sequence matching between two polypeptides or two nucleic acids. When a position in two compared sequences is occupied by the same base or amino acid monomer subunit (for example, a position in each of two DNA molecules is occupied by adenine, or a position in each of two polypeptides is occupied by lysine), then the molecules are identical at that position. The "identity" between two sequences, when expressed as a percentage, is a function of the number of matching positions shared by the two sequences divided by the number of positions being compared × 100. For example, if 6 out of 10 positions in one sequence match those in another sequence, then the two sequences are 60% identical. For example, the DNA sequences CTGACT and CAGGTT share 50% identity (3 out of 6 positions in one sequence match those in the other). Typically, comparisons are made when aligning two sequences to produce maximum identity. Such comparisons can be achieved by using, for example, the method described in Needleman et al. (1970) J. Mol. Biol. 48:443-453 that can be conveniently performed using computer programs such as the Align program (DNAstar, Inc.). The algorithm of E. Meyers and W. Miller (Comput. Appl Biosci., 4:11-17 (1988)) which has been integrated into the ALIGN program (version 2.0) can also be used to determine the percentage identity between two amino acid sequences using the PAM120 weight residue table, a 12 gap length penalty, and a 4 gap penalty. Furthermore, the Needleman and Wunsch (J Mol Biol.48:444-453(1970)) algorithm in the GAP program integrated into the GCG software package (available at http://www.gcg.com) can be used to determine the identity between two amino acid sequences using a Blossum 62 matrix or a PAM250 matrix and gap weights of 16, 14, 12, 10, 8, 6, or 4 and length weights of 1, 2, 3, 4, 5, or 6.

As defined herein, the terms "polypeptide", "protein", "peptide", and "amino acid sequence" are used interchangeably herein and refer to a polymer of amino acid residues of any length. The polymer can be a linear or branched chain, it can comprise modified amino acids or amino acid analogs, and it can be broken down by non-amino acid chemical moieties. The term further comprises amino acid polymers that have been naturally or artificially modified (e.g., disulfide bond formation, glycosylation, lipidation, acetylation, phosphorylation, or any other operation or modification, such as conjugation with a label or bioactive component). The term "peptide" comprises two or more naturally occurring or synthetic amino acids linked by covalent bonds (e.g., amide bonds).

When used herein, unless otherwise indicated, the site numbering of polypeptides or amino acid sequences, as is customary in the art, is in the order from the N-terminal to the C-terminal, for example, the first amino acid starting from the N-terminal of the polypeptide or amino acid sequence is the first amino acid, the second amino acid starting from the N-terminal of the polypeptide or amino acid sequence is the second amino acid, the third amino acid starting from the N-terminal of the polypeptide or amino acid sequence is the third amino acid, and so on. When used herein, unless otherwise indicated, the left-hand end of a polypeptide or amino acid sequence is the N-terminal, i.e., the amino terminal, and the right-hand end is the C-terminal, i.e., the carboxyl terminal.

In this disclosure, when referring to peptides or polypeptides, "amino acid" and "amino acid residue" have the same meaning, referring to the amino acid portion that remain after some groups are lost due to their participation in the formation of a linkage when amino acids are linked by chemical bonds. Such portion is the amino acid residue. The twenty common amino acids discussed herein are written in accordance with conventional usage. See, for example, Immunology-A Synthesis (2nd Edition, E.S. Golub and D.R. Gren, Eds., Sinauer Associates, Sunderland, Mass. (1991)), which is incorporated herein by reference. Amino acids herein are generally represented by single-letter and three-letter abbreviations known in the art. For example, alanine can be represented by A or Ala.

The term "amino acid" as used herein comprises natural amino acids, as well as other "non-proteinogenic" α-amino acids/non-natural amino acids commonly used in the field of peptide chemistry to prepare analogs of natural peptides.

Natural amino acids are glycine, alanine, valine, leucine, isoleucine, serine, methionine, threonine, phenylalanine, tyrosine, tryptophan, cysteine, proline, histidine, aspartic acid, asparagine, glutamic acid, glutamine, arginine, ornithine, and lysine. Examples of non-proteinogenic α-amino acids/non-natural amino acids include norleucine, norvaline, alloisoleucine, homoarginine, thioproline, dehydroproline, hydroxyproline (Hyp), homoserine, cyclohexylglycine (Chg), α-amino-n-butyric acid (Aba), cyclohexylalanine (Cha), aminophenylbutyric acid (Pba), phenylalanine with the phenyl moiety replaced by alkyl, alkoxy, halogen, or nitro groups, O-alkylated derivatives of serine, threonine, and tyrosine, S-alkylated cysteine, O-sulfate esters of tyrosine, and D-isomers of natural amino acids. In some embodiments of this document, non-natural amino acids comprise amino acids with olefin side chains, such as (*S*)-2-(4'-pentenyl)alanine (i.e. S5), (*R*)-2-(4'-pentenyl)alanine (i.e. R5), (*S*)-2-(7'-octenyl)alanine (i.e. S8), (*R*)-2-(7'-octenyl)alanine (i.e. R8), and 2-amino-2-(4'-pentenyl)-6-heptenoic acid (i.e. B5).

As used herein, the term "stereoisomer" means an isomer formed by having one or more stereoisomeric centers, and each stereoisomeric center may exist in an *R* or *S* configuration or a combination thereof. Similarly, the polypeptide described herein may have one or more double bonds, and each double bond may be in the form of an *E* (*trans*) or *Z* (*cis*) configuration or a combination thereof. A particular stereoisomer, regioisomer, diastereomer, enantiomer, or epimer should be understood to comprise all possible stereoisomers, regioisomers, diastereomers, enantiomers, or epimers and mixtures thereof. Hence, the polypeptide described herein comprises all configurations of stereoisomers, regioisomers, diastereomers, enantiomers or epimers, and mixtures thereof. The preferred purely structural isomers of the polypeptide of the present invention are enantiomers or diastereomers. Techniques for converting or maintaining a specific stereoisomer, as well as techniques for separating mixtures of stereoisomers, are well known in the art, and those skilled in the art can select the appropriate method for the specific circumstances.

As used herein, a stapled peptide refers to a polypeptide in which a covalent bond is formed between the side chains of two amino acids through an orthogonal reaction. A double-bridged stapled peptide refers to a polypeptide in which two independent covalent bonds are formed between the side chains of amino acids. Peptide stapling can be used to physically bind peptides to form or maintain a specific conformation (e.g., physically binding a peptide to maintain its original α-helical state). Stapling can enhance the pharmaceutical properties of peptides by helping to maintain the original structure required for peptides to interact with target molecules, increasing cell penetration, and/or protecting peptides from protein degradation.

As used herein, the term "functionally equivalent variant" refers to a polypeptide derived from the polypeptide sequence of the present invention by means of substitution, deletion or addition of amino acids, or a derivative polypeptide having a certain sequence identity to the polypeptide of the present invention, provided that the aforementioned derivative polypeptide maintains at least 50%, at least 80%, or at least 100% of the function based on the corresponding unmodified polypeptide. The functionally equivalent variant further comprises the polypeptide with enhanced functional activity compared to an unmodified polypeptide.

### Polypeptide, or stereoisomer, derivative, pharmaceutically acceptable salt, mixture, or functionally equivalent variant thereof

The present disclosure provides a polypeptide or the stereoisomer, derivative, pharmaceutically acceptable salt, mixture, or functionally equivalent variant thereof, wherein the polypeptide comprises an amino acid sequence represented by any one of the following general formulas (I)-(IV), or an amino acid sequence represented by any one of the general formulas (I)-(IV) having 1, 2, 3, 4, or 5 conservative substitutions:
X₁-X₂-X₃-X₄-X₅-X₆-X₇-X₈-X₉-X₁₀-X₁₁-X₁₂-X₁₃-X₁₄-W (I),
X₂-W-E-A-X₆-K-X₉-L-W-N-X₁₃-L-Q-Y-W (II),
G-X₂-E-A-L-X₆-Y-L-W-N-L-L-Q-Y-W (III),
G-W-E-A-L-K-Y-L-X₉-N-L-L-X₁₃-Y-W (IV),
wherein X₁ represents any amino acid, preferably G or W, or an amino acid residue having similar properties thereto; X₂, X₆, X₉, and X₁₃ are independently non-natural amino acids, wherein (X₂) and (X₆), and (X₉) and (X₁₃) are bonded to each other via a covalent bond linkage, respectively; X₃ represents any amino acid, preferably E or R, or an amino acid residue having similar properties thereto; X₄ represents any amino acid, preferably A or W, or an amino acid residue having similar properties thereto; X₅ represents any amino acid, preferably L or W, or an amino acid residue having similar properties thereto; X₇ represents any amino acid, preferably Y or R, or an amino acid residue having similar properties thereto; X₈ represents any amino acid, preferably L or W, or an amino acid residue having similar properties thereto; X₁₀ represents any amino acid, preferably N or R, or an amino acid residue having similar properties thereto; X₁₁ represents any amino acid, preferably L, W, or R, or an amino acid residue having similar properties thereto; X₁₂ represents any amino acid, preferably L or W, or an amino acid residue having similar properties thereto; X₁₄ represents any amino acid, preferably Y or R, or an amino acid residue having similar properties thereto; and the symbol "-" represents a peptide bond between amino acid residues. In some preferred embodiments, the polypeptide provided in the present disclosure has at least 40%, 50%, 60%, 70%, 71%, 72%, 73%, 80%, or 90% sequence identity to F9170 (SEQ ID NO: 5).

In a preferred embodiment, the non-natural amino acids comprised in the polypeptide disclosed herein, or the stereoisomer, derivative, pharmaceutically acceptable salt, mixture, or functionally equivalent variant of the polypeptide, are independently selected from amino acids with olefin side chains, such as (*S*)-2-(4'-pentenyl)alanine (i.e. S5), (*R*)-2-(4'-pentenyl)alanine (i.e. R5), (*S*)-2-(7'-octenyl)alanine (i.e. S8), (*R*)-2-(7'-octenyl)alanine (i.e. R8), and 2-amino-2-(4'-pentenyl)-6-heptenoic acid (i.e. B5); more preferably, the non-natural amino acid is (*S*)-2-(4'-pentenyl)alanine (i.e. S5). In a preferred embodiment, the covalent bond linkage formed between the non-natural amino acids comprises a disulfide bond, a hydrocarbon chain, an intramolecular amide bond, a thioether bond, or a covalent bond formed by an azide-alkyne cycloaddition reaction. More preferably, the covalent bond linkage is a covalent bond linkage formed by olefin metathesis, such as an all-hydrocarbon chain linking bridge.

In an embodiment, the polypeptide provided in the present disclosure comprises any one of the following (a)-(t) amino acid sequences: (a) an amino acid sequence having at least 65%, at least 70%, at least 75%, at least 80%, at least 85%, or at least 90% sequence identity to an amino acid sequence set forth in SEQ ID NO: 1, or an amino acid sequence having 1-5 conservative substitutions relative to the amino acid sequence set forth in SEQ ID NO: 1, preferably the amino acid sequence set forth in SEQ ID NO: 1; (b) an amino acid sequence having at least 65%, at least 70%, at least 75%, at least 80%, at least 85%, or at least 90% sequence identity to an amino acid sequence set forth in SEQ ID NO: 2, or an amino acid sequence having 1-5 conservative substitutions relative to the amino acid sequence set forth in SEQ ID NO: 2, preferably the amino acid sequence A(S5)EAL(S5)YL(S5)NLL(S5)YW set forth in SEQ ID NO: 2, wherein the first and second S5 residues are covalently linked, and the third and fourth S5 residues are covalently linked; (c) an amino acid sequence having at least 65%, at least 70%, at least 75%, at least 80%, at least 85%, or at least 90% sequence identity to an amino acid sequence set forth in SEQ ID NO: 3, or an amino acid sequence having 1-5 conservative substitutions relative to the amino acid sequence set forth in SEQ ID NO: 3, preferably the amino acid sequence set forth in SEQ ID NO: 3; (d) an amino acid sequence having at least 65%, at least 70%, at least 75%, at least 80%, at least 85%, or at least 90% sequence identity to an amino acid sequence set forth in SEQ ID NO: 10, or an amino acid sequence having 1-5 conservative substitutions relative to the amino acid sequence set forth in SEQ ID NO: 10, preferably the amino acid sequence set forth in SEQ ID NO: 10; (e) an amino acid sequence having at least 65%, at least 70%, at least 75%, at least 80%, at least 85%, or at least 90% sequence identity to an amino acid sequence set forth in SEQ ID NO: 11, or an amino acid sequence having 1-5 conservative substitutions relative to the amino acid sequence set forth in SEQ ID NO: 11, preferably the amino acid sequence set forth in SEQ ID NO: 11; (f) an amino acid sequence having at least 65%, at least 70%, at least 75%, at least 80%, at least 85%, or at least 90% sequence identity to an amino acid sequence set forth in SEQ ID NO: 12, or an amino acid sequence having 1-5 conservative substitutions relative to the amino acid sequence set forth in SEQ ID NO: 12, preferably the amino acid sequence set forth in SEQ ID NO: 12; (g) an amino acid sequence having at least 65%, at least 70%, at least 75%, at least 80%, at least 85%, or at least 90% sequence identity to an amino acid sequence set forth in SEQ ID NO: 13, or an amino acid sequence having 1-5 conservative substitutions relative to the amino acid sequence set forth in SEQ ID NO: 13, preferably the amino acid sequence set forth in SEQ ID NO: 13; (h) an amino acid sequence having at least 65%, at least 70%, at least 75%, at least 80%, at least 85%, or at least 90% sequence identity to an amino acid sequence set forth in SEQ ID NO: 14, or an amino acid sequence having 1-5 conservative substitutions relative to the amino acid sequence set forth in SEQ ID NO: 14, preferably the amino acid sequence set forth in SEQ ID NO: 14; (i) an amino acid sequence having at least 65%, at least 70%, at least 75%, at least 80%, at least 85%, or at least 90% sequence identity to an amino acid sequence set forth in SEQ ID NO: 15, or an amino acid sequence having 1-5 conservative substitutions relative to the amino acid sequence set forth in SEQ ID NO: 15, preferably the amino acid sequence set forth in SEQ ID NO: 15; (j) an amino acid sequence having at least 65%, at least 70%, at least 75%, at least 80%, at least 85%, or at least 90% sequence identity to an amino acid sequence set forth in SEQ ID NO: 16, or an amino acid sequence having 1-5 conservative substitutions relative to the amino acid sequence set forth in SEQ ID NO: 16, preferably the amino acid sequence set forth in SEQ ID NO: 16; (k) an amino acid sequence having at least 65%, at least 70%, at least 75%, at least 80%, at least 85%, or at least 90% sequence identity to an amino acid sequence set forth in SEQ ID NO: 17, or an amino acid sequence having 1-5 conservative substitutions relative to the amino acid sequence set forth in SEQ ID NO: 17, preferably the amino acid sequence set forth in SEQ ID NO: 17; (l) an amino acid sequence having at least 65%, at least 70%, at least 75%, at least 80%, at least 85%, or at least 90% sequence identity to an amino acid sequence set forth in SEQ ID NO: 18, or an amino acid sequence having 1-5 conservative substitutions relative to the amino acid sequence set forth in SEQ ID NO: 18, preferably the amino acid sequence set forth in SEQ ID NO: 18; (m) an amino acid sequence having at least 65%, at least 70%, at least 75%, at least 80%, at least 85%, or at least 90% sequence identity to an amino acid sequence set forth in SEQ ID NO: 19, or an amino acid sequence having 1-5 conservative substitutions relative to the amino acid sequence set forth in SEQ ID NO: 19, preferably the amino acid sequence set forth in SEQ ID NO: 19; (n) an amino acid sequence having at least 65%, at least 70%, at least 75%, at least 80%, at least 85%, or at least 90% sequence identity to an amino acid sequence set forth in SEQ ID NO: 20, or an amino acid sequence having 1-5 conservative substitutions relative to the amino acid sequence set forth in SEQ ID NO: 20, preferably the amino acid sequence set forth in SEQ ID NO: 20; (o) an amino acid sequence having at least 65%, at least 70%, at least 75%, at least 80%, at least 85%, or at least 90% sequence identity to an amino acid sequence set forth in SEQ ID NO: 21, or an amino acid sequence having 1-5 conservative substitutions relative to the amino acid sequence set forth in SEQ ID NO: 21, preferably the amino acid sequence set forth in SEQ ID NO: 21; (p) an amino acid sequence having at least 65%, at least 70%, at least 75%, at least 80%, at least 85%, or at least 90% sequence identity to an amino acid sequence set forth in SEQ ID NO: 22, or an amino acid sequence having 1-5 conservative substitutions relative to the amino acid sequence set forth in SEQ ID NO: 22, preferably the amino acid sequence set forth in SEQ ID NO: 22; (q) an amino acid sequence having at least 65%, at least 70%, at least 75%, at least 80%, at least 85%, or at least 90% sequence identity to an amino acid sequence set forth in SEQ ID NO: 23, or an amino acid sequence having 1-5 conservative substitutions relative to the amino acid sequence set forth in SEQ ID NO: 23, preferably the amino acid sequence set forth in SEQ ID NO: 23; (r) an amino acid sequence having at least 65%, at least 70%, at least 75%, at least 80%, at least 85%, or at least 90% sequence identity to an amino acid sequence set forth in SEQ ID NO: 24, or an amino acid sequence having 1-5 conservative substitutions relative to the amino acid sequence set forth in SEQ ID NO: 24, preferably the amino acid sequence set forth in SEQ ID NO: 24; (s) an amino acid sequence having at least 65%, at least 70%, at least 75%, at least 80%, at least 85%, or at least 90% sequence identity to an amino acid sequence set forth in SEQ ID NO: 8, or an amino acid sequence having 1-5 conservative substitutions relative to the amino acid sequence set forth in SEQ ID NO: 8, preferably the amino acid sequence set forth in SEQ ID NO: 8; and (t) an amino acid sequence having at least 65%, at least 70%, at least 75%, at least 80%, at least 85%, or at least 90% sequence identity to an amino acid sequence set forth in SEQ ID NO: 9, or an amino acid sequence having 1-5 conservative substitutions relative to the amino acid sequence set forth in SEQ ID NO: 9, preferably the amino acid sequence set forth in SEQ ID NO: 9.

In an embodiment, the polypeptide provided in the present disclosure, or the stereoisomer, derivative, pharmaceutically acceptable salt, mixture, or functionally equivalent variant of the polypeptide has one or more of the following advantages: simple synthesis, low synthesis cost, oral administration capability or oral administration potential, high antiviral activity for inhibiting HIV replication, high activity for lysing HIV viral particles, high activity for inactivating free HIV particles, long-acting effect, effective entry into an HIV anatomical reservoir, and good biosafety. In an embodiment, compared with F9170, the polypeptide provided in the present disclosure, or the stereoisomer, derivative, pharmaceutically acceptable salt, mixture, or functionally equivalent variant of the polypeptide has the advantages of improved half-life, oral administration capability or oral administration potential, and/or effective entry into an HIV anatomical reservoir.

As used herein, the "derivative" of the polypeptide refers to a product obtained by amino acid modification, conservative amino acid substitution, and/or substitution of hydrogen in amino acid residues based on the polypeptide mentioned herein.

When used herein, the types of the term "amino acid modification" comprise, but are not limited to, N-terminal modification, C-terminal modification, and side chain modification. "Amino acid modification" methods comprise, but are not limited to, hydroxylation, carboxylation, alkylation, acylation, phosphorylation, sulfonation, amidation, aldehydation, alcoholylation, mercaptoethylamination, esterification, and glycosylation.

When used herein, "conservative amino acid substitution" can generally be described as the substitution of one amino acid residue by another amino acid with a similar chemical structure and/or similar chemical properties, and an amino acid substitution having little effect on the function, activity or other biological properties of the peptide. Such conservative amino acid substitutions are well known in the art. Such conservative substitutions can be, for example, the substitution of one amino acid in the following groups (a)-(e) by another amino acid in the same group: (a) small aliphatic, nonpolar, or weakly polar amino acid residues: Ala, Ser, Thr, Pro, and Gly; (b) negatively charged polar amino acid residues and their amides: Asp, Asn, Glu, and Gin; (c) positively charged polar amino acid residues: His, Arg, and Lys; (d) large aliphatic, nonpolar amino acid residues: Met, Leu, Ile, Val, and Cys; (e) aromatic amino acid residues: Phe, Tyr, and Trp. Conservative amino acid substitution can be the substitution of an amino acid residue by an amino acid residue with a similar side chain. Families of amino acid residues with similar side chains have been defined in the art, and comprise basic side chains (e.g., lysine, arginine, and histidine), acidic side chains (e.g., aspartic acid, and glutamic acid), uncharged polar side chains (e.g., glycine, asparagine, glutamine, serine, threonine, tyrosine, and cysteine), nonpolar side chains (e.g., alanine, valine, leucine, isoleucine, proline, phenylalanine, methionine, and tryptophan), β-branched side chains (e.g., threonine, valine, and isoleucine), and aromatic side chains (e.g., tyrosine, phenylalanine, tryptophan, and histidine). Conservative modifications can be selected, for example, based on the similarity of polarity, charge, solubility, hydrophobicity, hydrophilicity, and/or the amphiphilic properties of the residues involved.

As used herein, substitutions between amino acid residues having similar properties have little effect on the function, activity, or other biological properties of the peptide.

When used herein, the substitution of hydrogen in an amino acid residue can be the substitution of hydrogen in the amino acid residue by any conventional substituent known in the art, including, but not limited to, alkyl, alkenyl, alkynyl, aromatic, alkoxy, carboxyl, aldehyde, carbonyl, hydroxyl, halogen, oxy, acyl, sulfonic acid, amino, mercapto, or nitro substituents.

In an embodiment, the derivative of the polypeptide provided in the present disclosure is selected from a derivative obtained by maleimide modification of the polypeptide, a derivative obtained by attaching an amino-terminal protecting group to the N-terminus and/or a carboxyl-terminal protecting group to the C-terminus, a derivative obtained by protein or polyethylene glycol modification of the polypeptide, a derivative obtained by attaching an oligopeptide, a lipophilic group, or cholesterol to the N-terminus and/or the C-terminus, or a derivative obtained by substituting one or more amino acid residues with D-amino acids, artificially modified amino acids, or naturally occurring rare amino acids.

In an embodiment, the amino-terminal protecting group provided in the present invention is selected from: H, a polyethylene glycol-derived polymer, an unsubstituted or substituted acyclic aliphatic group, an unsubstituted or substituted alicyclic group, an unsubstituted or substituted heterocyclic group, an unsubstituted or substituted heteroarylalkyl group, an unsubstituted or substituted aryl group, an unsubstituted or substituted aralkyl group, and R5-CO-, wherein R5 is selected from: H, an unsubstituted or substituted acyclic aliphatic group, an unsubstituted or substituted alicyclic group, an unsubstituted or substituted aryl group, an unsubstituted or substituted aralkyl group, an unsubstituted or substituted heterocyclic group, and an unsubstituted or substituted heteroarylalkyl group. Preferably, the amino-terminal protecting group is selected from: H, acetyl (Ac), lauroyl, myristoyl, and palmitoyl.

In an embodiment, the carboxyl-terminal protecting group provided in the present invention is selected from: -NR3R4, -OR3, and -SR3, wherein R3 and R4 are independently selected from: H, a polyethylene glycol-derived polymer, an unsubstituted or substituted acyclic aliphatic group, an unsubstituted or substituted alicyclic group, an unsubstituted or substituted heterocyclic group, an unsubstituted or substituted heteroarylalkyl group, an unsubstituted or substituted aryl group, and an unsubstituted or substituted aralkyl group. Preferably, the carboxyl-terminal protecting group is selected from: -NR3R4 and -OR3, wherein R3 and R4 are independently selected from H, methyl, ethyl, hexyl, dodecyl, and hexadecyl.

### Pharmaceutical composition, drug conjugate, drug complex, fusion protein

The present disclosure provides a pharmaceutical composition comprising at least the polypeptide disclosed herein, or the stereoisomer, derivative, pharmaceutically acceptable salt, mixture, or functionally equivalent variant of the polypeptide, and a pharmaceutically acceptable carrier or excipient. In an embodiment, the pharmaceutical composition may optionally further comprise one or more additional HIV therapeutic agents or HIV latency-reversing agents.

HIV latency-reversing agents comprise histone deacetylase inhibitors (HDACi), bromodomain (BRD) protein inhibitors, protein kinase C (PKC) activators, positive transcription elongation factor b (p-TEFb) activators, DNA methyltransferase inhibitors (DNMTi), and cytokines. Anti-HIV drugs or HIV therapeutic agents comprise, but are not limited to, reverse transcriptase inhibitors, protease inhibitors, entry inhibitors, integrase inhibitors, and maturation inhibitors. The reverse transcriptase inhibitors may be one or more of AZT, 3TC, ddI, d4T, ddT, TDF, Abacavir, Nevirapine Efavirenz, Delavirdine, etc.; the protease inhibitors may be one or more of Saquinavir mesylate, Indinavir, Ritonavir, Amprenavir, Kaletra, Nelfinavir mesylate, etc.; the entry inhibitors may be one or more of Maraviroc, TAK-779, T20, T2635, sifuvirtide, VIRIP, etc.; the integrase inhibitors may be Raltegravir, etc.; the maturation inhibitors may be GSK3640254, Bevirimat, GSK3532795, GSK3640254, PF-46396, etc.; and the integrase inhibitors may be dolutegravir (DTG), elvitegravir (EVG), bictegravir (BIC), cabotegravir (CAB), etc.

HIV therapeutic agents comprise, but are not limited to, abacavir, emtricitabine, lamivudine, tenofovir disoproxil fumarate, zidovudine, doravirine, efavirenz, etravirine, nevirapine, rilpivirine, atazanavir, darunavir, fosamprenavir, ritonavir, saquinavir, tipranavir, enfuvirtide, maraviroc, dolutegravir, raltegravir, ibalizumab, cobicistat, bictegravir, telarfe, rilpivirine, elvitegravir, or lopinavir. As used herein, the term "pharmaceutical composition" means a mixture of the polypeptide disclosed herein, or the stereoisomer, derivative, pharmaceutically acceptable salt, mixture, or functionally equivalent variant thereof with other chemical components such as a carrier, stabilizer, diluent, dispersant, suspending agent, thickener and/or excipient. The pharmaceutical composition facilitates the administration of the polypeptide, or the stereoisomer, derivative, pharmaceutically acceptable salt, mixture, or functionally equivalent variant of the polypeptide to a living organism. There are a variety of ways in which the polypeptide, or the stereoisomer, derivative, pharmaceutically acceptable salt, mixture, or functionally equivalent variant of the polypeptide can be administered, including, but not limited to, subcutaneous injection, intramuscular injection, intravenous injection, intraperitoneal injection, intrathecal injection, oral administration, transdermal, transpulmonary, ocular and topical administration.

In the present application, the pharmaceutical composition may be configured as dosage forms suitable for administration to a subject via the desired route of administration. The dosage forms comprise, but are not limited to, tablets, capsules, caplets, pills, lozenges, powders, syrups, brewing agents, suspensions, solutions, emulsions, transdermal patches, suppositories, inhalants, creams, ointments, lotions, pastes, sprays, lyophilized products, injections, and gels.

When used herein, the term "pharmaceutically acceptable salt" comprises both acid addition salts and base addition salts. Appropriate acid addition salts are formed from acids that form non-toxic salts. Examples thereof comprise, but are not limited to: acetates, adipates, aspartates, benzoates, benzene sulfonates, bicarbonates/carbonates, hydrogen sulfates/sulfates, borates, camphor sulfonates, citrates, cyclohexylamine sulfonates, ethanedisulfonates, formates, fumarates, glucoheptonates, gluconate, glucuronates, hexafluorophosphates, 2-(4-hydroxybenzyl)benzoates, hydrochlorides/chlorides, hydrobromides/bromides, hydroiodides/iodides, 2-hydroxyethanesulfonates, lactates, malates, maleates, malonates, methanesulfonates, methyl sulfates, naphthenates, 2-naphthalenesulfonates, nicotinates, nitrates, orotates, oxalates, hexadecanoates, phosphates/hydrogen phosphates/dihydrogen phosphates, pyroglutamates, glucarates, stearates, salicylates, tannates, tartrates, toluenesulfonates, and trifluoroacetates. Appropriate base addition salts are formed from bases that form non-toxic salts. Examples thereof comprise, but are not limited to: aluminum, arginine, calcium, choline, diethylamine, diethanolamine, glycine, lysine, magnesium, meglumine, ethanolamine, potassium, sodium, tromethamine, and zinc salts. It can also form hemi-salts of acids and bases, such as hemisulphates and hemicalcium salts. For a review of suitable salts, see Handbook of Pharmaceutical Salts: Properties, Selection and Use by Stahl and Wermuth (Wiley-VCH, 2002).

The term "pharmaceutically acceptable carrier" comprises pharmaceutically acceptable materials, compositions, or carriers, such as liquid or solid fillers, diluents, excipients, solvents, or encapsulating materials, relating to carrying or delivering within a subject the polypeptide of the present disclosure, or the stereoisomer, derivative, pharmaceutically acceptable salt, mixture, or functionally equivalent variant of the polypeptide, or carrying or delivering the polypeptide of the present disclosure, or the stereoisomer, derivative, pharmaceutically acceptable salt, mixture, or functionally equivalent variant of the polypeptide, to a subject so that they can perform their intended functions. Each salt or carrier must be "acceptable" in the sense of compatibility with other components of the formulation and must not be harmful to the subjects. Some examples of materials that can be used as pharmaceutically acceptable carriers comprise: sugars, such as lactose, glucose, and sucrose; starches, such as corn starch and potato starch; cellulose and derivatives thereof, such as sodium carboxymethyl cellulose, ethyl cellulose, and cellulose acetate; powdered tragacanth gum; malt; gelatin; talc; excipients, such as cocoa butter and suppository waxes; oils, such as peanut oil, cottonseed oil, safflower oil, sesame oil, olive oil, corn oil, and soybean oil; glycols, such as propylene glycol; polyols, such as glycerol, sorbitol, mannitol, and polyethylene glycol; esters, such as ethyl oleate and ethyl laurate; agar; buffers, such as magnesium hydroxide and aluminum hydroxide; alginate; pyrogen-free water; isotonic saline; Ringer's solution; ethanol; phosphate buffer solution; diluents; granulating agents; lubricants; binders; disintegrants; wetting agents; emulsifiers; colorants; release agents; coating agents; sweeteners; flavoring agents; aromatizing agents; preservatives; antioxidants; plasticizers; gelling agents; thickeners; hardeners; setting agents; suspending agents; surfactants; humectants; carriers; stabilizers; and other non-toxic, compatible substances used in pharmaceutical formulations, or any combination thereof.

The present disclosure provides a drug conjugate comprising at least the polypeptide disclosed herein, or the stereoisomer, derivative, pharmaceutically acceptable salt, mixture, or functionally equivalent variant of the polypeptide. In an embodiment, the drug conjugate may optionally comprise a carrier protein, such as serum albumin, immunoglobulin, ferritin, transferrin, α-2-macroglobulin, thyroxine-binding protein, or steroid-binding protein. In an embodiment, the drug conjugate may optionally comprise one or more additional HIV therapeutic agents or HIV latency-reversing agents. In an embodiment, the drug conjugate is formed by linking the polypeptide disclosed herein, or the stereoisomer, derivative, pharmaceutically acceptable salt, mixture, or functionally equivalent variant of the polypeptide, to a carrier protein via a linker. In an embodiment, the drug conjugate is formed by linking the polypeptide disclosed herein, or the stereoisomer, derivative, pharmaceutically acceptable salt, mixture, or functionally equivalent variant of the polypeptide, to another HIV therapeutic agent or an HIV latency-reversing agent via a linker. The linker can be a cleavable linker or non-cleavable linker.

The present disclosure provides a drug complex comprising at least the polypeptide disclosed herein, or the stereoisomer, derivative, pharmaceutically acceptable salt, mixture, or functionally equivalent variant of the polypeptide. In an embodiment, the drug complex may optionally comprise a carrier protein, such as serum albumin, immunoglobulin, ferritin, transferrin, α-2-macroglobulin, thyroxine-binding protein, or steroid-binding protein. In an embodiment, the drug complex may optionally comprise one or more additional HIV therapeutic agents or HIV latency-reversing agents. In an embodiment, the drug complex is formed by covalently attaching the polypeptide disclosed herein, or the stereoisomer, derivative, pharmaceutically acceptable salt, mixture, or functionally equivalent variant of the polypeptide, to a carrier protein. In an embodiment, the drug complex is formed by covalently attaching the polypeptide disclosed herein, or the stereoisomer, derivative, pharmaceutically acceptable salt, mixture, or functionally equivalent variant of the polypeptide, to another HIV therapeutic agent or an HIV latency-reversing agent.

The present disclosure provides a fusion protein comprising at least the polypeptide disclosed herein, or the stereoisomer, derivative, pharmaceutically acceptable salt, mixture, or functionally equivalent variant of the polypeptide. In an embodiment, the fusion protein may optionally comprise one or more additional proteins for treating or preventing HIV infection, such as an antibody against an intracellular domain of an HIV envelope protein, preferably an antibody against LLP1-GQ, such as the LLP1-GQ antibody set forth in SEQ ID NO: 4 (GACRAIRHIPRRIRQ). In an embodiment, the polypeptide disclosed herein, or the stereoisomer, derivative, pharmaceutically acceptable salt, mixture, or functionally equivalent variant of the polypeptide comprised by the fusion protein is linked, by covalent bonds, to one or more additional proteins used for treating or preventing HIV infection.

As described herein, LLP1-GQ is the domain of lentiviral lytic peptide-1 (LLP-1), which is amino acids 828-842 on LLP1, and has the sequence set forth in GACRAIRHIPRRIRQ (SEQ ID NO: 4).

In an embodiment, the pharmaceutical composition, drug conjugate, drug complex, or fusion protein disclosed herein can be independently formulated into dosage forms comprising tablets, pills, powders, suppositories, solutions, suspensions, emulsions, granules, tinctures, capsules, transdermal preparations, aerosols, effervescent tablets, drops, and lyophilized powders.

In an embodiment, the pharmaceutical composition, drug conjugate, drug complex, or fusion protein disclosed herein are administered in a variety of ways, including, but not limited to, subcutaneous injection, intramuscular injection, intravenous injection, intraperitoneal injection, intrathecal injection, oral administration, transdermal, transpulmonary, ocular, and topical administration.

### Use and Methods

The present disclosure provides the use of the polypeptide disclosed herein, or the stereoisomer, derivative, pharmaceutically acceptable salt, mixture, or functionally equivalent variant of the polypeptide, in the preparation of a medicament for treating or preventing diseases caused by HIV infection. In an embodiment, the dosage forms of the medicament comprise tablets, pills, powders, suppositories, solutions, suspensions, emulsions, granules, tinctures, capsules, transdermal preparations, aerosols, effervescent tablets, drops, and lyophilized powders. In an embodiment, the medicament is administered in a variety of ways, including, but not limited to, subcutaneous injection, intramuscular injection, intravenous injection, intraperitoneal injection, intrathecal injection, oral administration, transdermal, transpulmonary, ocular, and topical administration.

The present disclosure provides the use of the polypeptide disclosed herein, or the stereoisomer, derivative, pharmaceutically acceptable salt, mixture, or functionally equivalent variant of the polypeptide, as a medicament for treating or preventing diseases caused by HIV infection.

The present disclosure provides a method for treating or preventing diseases caused by HIV infection, comprising administering to a subject in need thereof a therapeutically effective amount of the polypeptide disclosed herein, or the stereoisomer, derivative, pharmaceutically acceptable salt, mixture, or functionally equivalent variant of the polypeptide, or the pharmaceutical composition, drug conjugate, drug complex, or fusion protein disclosed herein. In an embodiment, the polypeptide disclosed herein, or the stereoisomer, derivative, pharmaceutically acceptable salt, mixture, or functionally equivalent variant of the polypeptide, the pharmaceutical composition, drug conjugate, drug complex, or fusion protein disclosed herein is administered in the following ways: injection administration (including, but not limited to, subcutaneous injection, intravenous injection, intramuscular injection, intraperitoneal injection, intracisternal injection, etc.), cavity administration (including, but not limited to, digestive tract administration, reproductive tract administration, such as oral, gastrointestinal, rectal, vaginal, or sublingual administration, etc.), respiratory tract administration (including, but not limited to, nasal, transpulmonary administration), mucosal administration, or surface administration, preferably injection administration.

When used herein, the term "subject" comprises animals such as vertebrates, preferably mammals such as dogs, cats, pigs, cows, sheep, horses, rodents (e.g., mice, rats, or guinea pigs) or primates (e.g., gorillas, chimpanzees, and humans).

When used herein, the term "treatment" means to alleviate or improve a disease or disorder (i.e., to slow or stop the development of a disease or at least one clinical symptom); or to alleviate or improve at least one physical parameter or biomarker associated with the disease or disorder.

When used herein, the term "therapeutically effective amount" refers to the amount that results in benefit or treatment of disease in a corresponding subject who did not receive the amount, but is low enough within the range of reasonable medical judgment to avoid serious side effects. The therapeutically effective amount of the polypeptide, or the stereoisomer, derivative, pharmaceutically acceptable salt, mixture, or functionally equivalent variant of the polypeptide, the pharmaceutical composition, drug conjugate, drug complex, or fusion protein described herein will vary depending on factors such as the selected polypeptide, or the stereoisomer, derivative, pharmaceutically acceptable salt, mixture, or functionally equivalent variant of the polypeptide, or the pharmaceutical composition, drug conjugate, drug complex, or fusion protein; the route of administration; the severity of the disease being treated; the age, body type, weight, and physical condition of the patient being treated; the patient's medical history; the duration of treatment; the nature of concurrent treatments; and the desired therapeutic effect, but can still be determined by those skilled in the art in a conventional manner.

As used herein, the term "prevention" refers to methods implemented to block, reduce, inhibit, prevent and/or delay the occurrence of a disease or condition or symptom (e.g., respiratory diseases and symptoms, infections or autoimmune diseases) in a subject, or methods to reduce the incidence of infectious diseases in said subject.

The present disclosure provides methods for use in vitro for any one or more of the following (a)-(e):
(a) inhibiting HIV infection and/or replication;
(b) inactivating free HIV viral particles;
(c) preparing a formulation having in vitro HIV infection-inhibitory activity;
(d) eliminating viruses in an HIV reservoir or reducing the number of viruses in the HIV reservoir; and
(e) lysing HIV viral particles.

The method comprises using the polypeptide disclosed herein, or the stereoisomer, derivative, pharmaceutically acceptable salt, mixture, or functionally equivalent variant of the polypeptide.

The present disclosure provides for the use in vitro of the polypeptide disclosed herein, or the stereoisomer, derivative, pharmaceutically acceptable salt, mixture, or functionally equivalent variant of the polypeptide, in any one or more of the following (a)-(e):
(a) inhibiting HIV infection and/or replication;
(b) inactivating free HIV viral particles;
(c) preparing a formulation having in vitro HIV infection-inhibitory activity;
(d) eliminating viruses in an HIV reservoir or reducing the number of viruses in the HIV reservoir; and
(e) lysing HIV viral particles.

The present disclosure provides a method for producing the polypeptide disclosed herein, or the stereoisomer, derivative, pharmaceutically acceptable salt, mixture, or functionally equivalent variant of the polypeptide, or the fusion protein disclosed herein, comprising chemically synthesizing the polypeptide, or the stereoisomer, derivative, pharmaceutically acceptable salt, mixture, or functionally equivalent variant of the polypeptide, or the fusion protein. In an embodiment, the chemical method may be a solid-phase (e.g., Fmoc solid-phase synthesis) or liquid-phase synthesis. In an embodiment, the method used herein for producing the polypeptide disclosed herein, or the stereoisomer, derivative, pharmaceutically acceptable salt, mixture, or functionally equivalent variant of the polypeptide, or the fusion protein disclosed herein, are methods commonly used in the art. In some embodiments, the polypeptide of the present invention can be chemically synthesized. For example, peptides can be synthesized using solid-phase methods (Stewart, J.M. and Young, J.D., "Solid Phase Peptide Synthesis, 2nd Edition", (1984), Pierce Chemical Company, Rockford, Illinois (1984);

Bodanszky M. and Bodanszky A., "The practice of Peptide Synthesis", (1994), Springer Verlag, Berlin; Lloyd-Williams P et al. / 'Chemical Approaches to the Synthesis of Peptides and Proteins', (1997), CRC, Boca Raton, FL, USA), Synthesis in solution, enzymatic synthesis (Kullmann W. "Proteases as catalysts for enzymic syntheses of opioid peptides", (1980), J. Biol. Chem., 255(17), 8234-8238) or any combination thereof.

The various embodiments described above with respect to the polypeptide of the present disclosure, or the stereoisomer, derivative, pharmaceutically acceptable salt, mixture, or functionally equivalent variant of the polypeptide, are also applicable to the pharmaceutical composition, drug complex, drug conjugate, fusion protein, use and method of the present disclosure (provided they are not inherently contradictory to one another, and all embodiments formed therefrom are considered part of the present disclosure.

### Examples

The following is a description of exemplary embodiments of the present application in conjunction with the drawings, comprising various details of the embodiments of the present application to facilitate understanding. It is to be understood that they are considered to be merely exemplary and are by no means intended to limit the scope of protection of the present application. The scope of protection of the present application is limited only by the claims. Accordingly, one of ordinary skill in the art should recognize that various changes and modifications can be made to the embodiments described here without departing from the scope of the present application. Similarly, for the sake of clarity and conciseness, descriptions of well-known functions and structures are omitted in the following description.

Unless otherwise specified, the reagents and instruments used in the following embodiments are all commercially available conventional products. Unless otherwise specified, the experiments were performed under conventional conditions or conditions recommended by the manufacturer.

### Example 1. Synthesis and Purification of Polypeptides

The polypeptides D26, D26W1, D26W4, D26W5, D26W8, D26W11, D26W12, D26R3, D26R7, D26R10, D26R11, D26R14, D26-2R, D26-3R, D26-4R, D26-5R, D1, F2, and F6 provided in the present disclosure were synthesized using a CEM Libery Blue microwave peptide synthesizer by the Fmoc solid-phase peptide synthesis(SPSS). The solid phase carrier used was Rink Amide Resin with a resin loading of 0.53 mmol/g. The condensation reagent was Oxyma/DIC, and the deprotection reagent was a 20% piperidine solution. Washing was performed three times with DMF solution after each condensation reaction.

The ratio of amino acid amount to Rink Amide Resin amount was 6 : 1. The following amino acids were used: Fmoc-Ala-OH, Fmoc-Asn(Trt)-OH, Fmoc-Gln(Trt)-OH, Fmoc-Glu(tBu)-OH, Fmoc-Gly-OH, Fmoc-Leu-OH, Fmoc-Lys(Boc)-OH, Fmoc-Trp(Boc)-OH, Fmoc-Tyr(tBu)-OH, Fmoc-Arg(Pbf)-OH, and the non-natural amino acid S5. Amino acid coupling was performed according to the polypeptide sequence and instrument operating procedures. If the polypeptide sequence contained isoleucine or leucine, the isoleucine, leucine, and amino acids following the amino acids thereof were condensed twice by default. The special amino acids that existed, such as S5 in the sequence under experiment, were condensed twice by default. After the amino acid linkage was complete, the peptide-resin was removed, the terminal Fmoc was removed, and then acetic anhydride/DIEA = 1/1 was added and stirred for 30 min to complete acetylation.

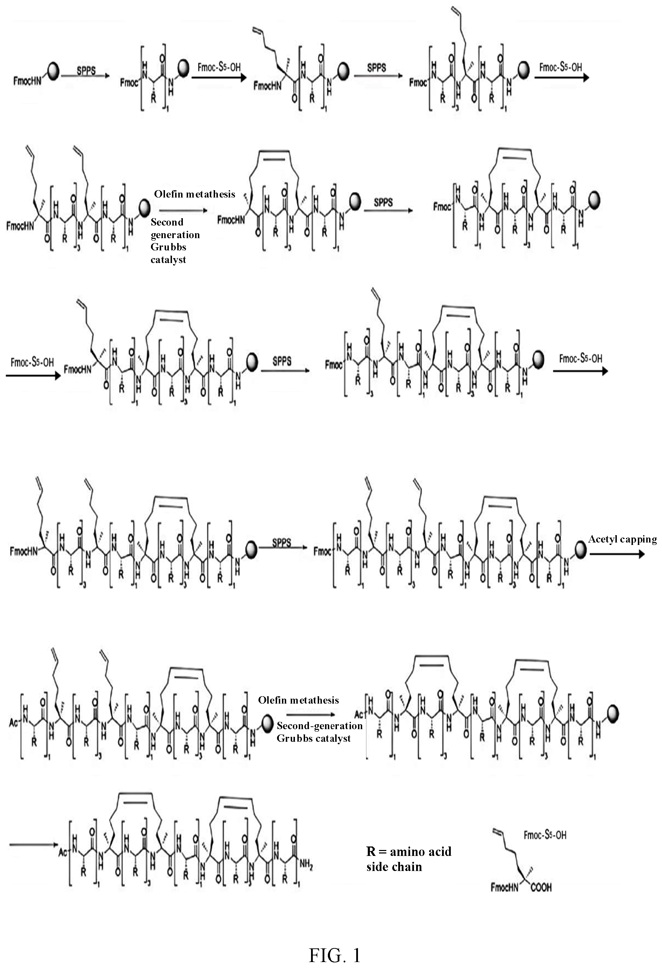

When F2 and F6 were single bridged, the acetylated peptide-resin underwent olefin metathesis without deprotection to form a hydrocarbon chain linking bridge. When D26 and D1 double-bridged hydrocarbon chain stapled peptides were synthesized, the peptide resin after synthesizing the first two S5 amino acids was removed, and the olefin metathesis was repeated without deprotection to perform the first "stapling". The remaining amino acids were continued to be coupled. After solid-phase synthesis was complete, the deprotection and acetylation were repeated and the second "stapling" operation was performed on the peptide resin. The specific process is shown in FIG. 1.

The polypeptide with the completed sequence was then lysed. 10 mL of lysis buffer (trifluoroacetic acid/ethylenedithiol/water/anisole/cresol= 8.25/0.25/0.5/0.5/0.5, v/v) was added to every 0.1 mmol peptide-resin. The mixture was then subjected to ice bathing for 30 min and stirred at room temperature for 3 h. Finally, anhydrous diethyl ether was added to precipitate the polypeptides, followed by washing and dissolution with a mixture of acetonitrile and water. The solution was then transferred to centrifuge tubes and lyophilized to obtain crude peptides.

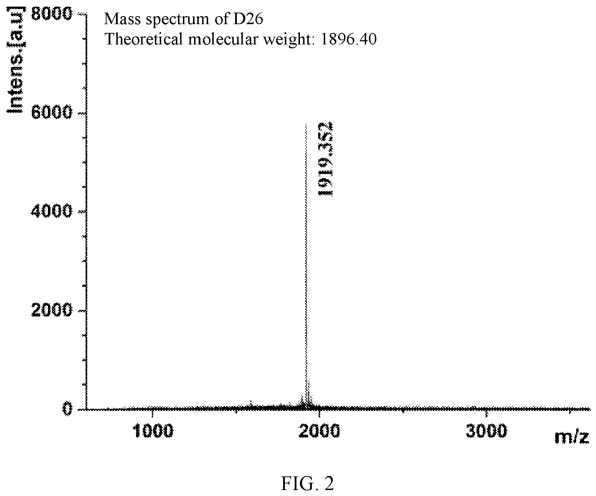

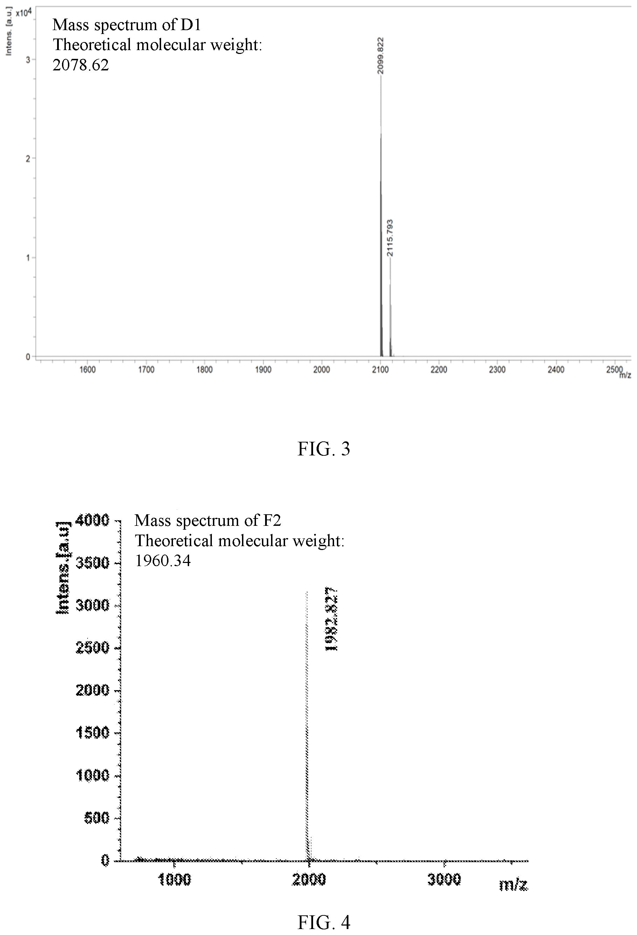

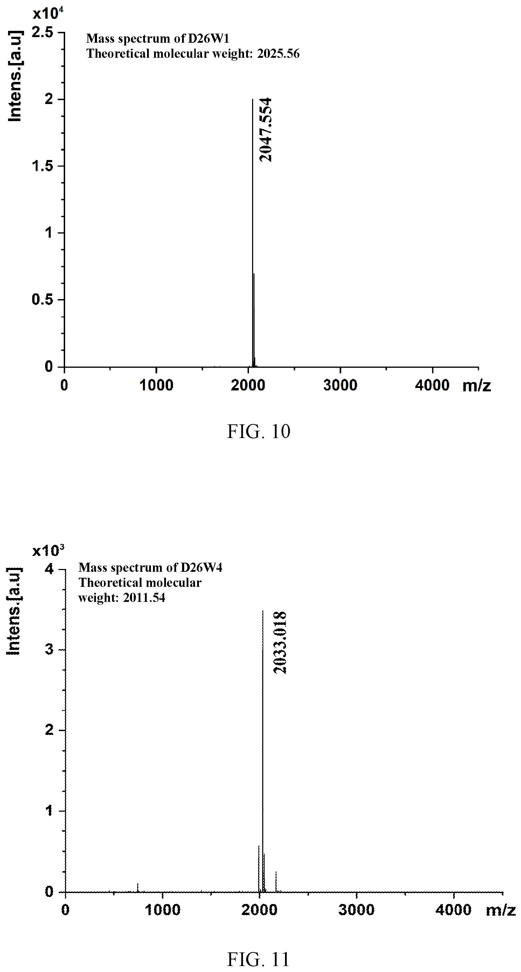

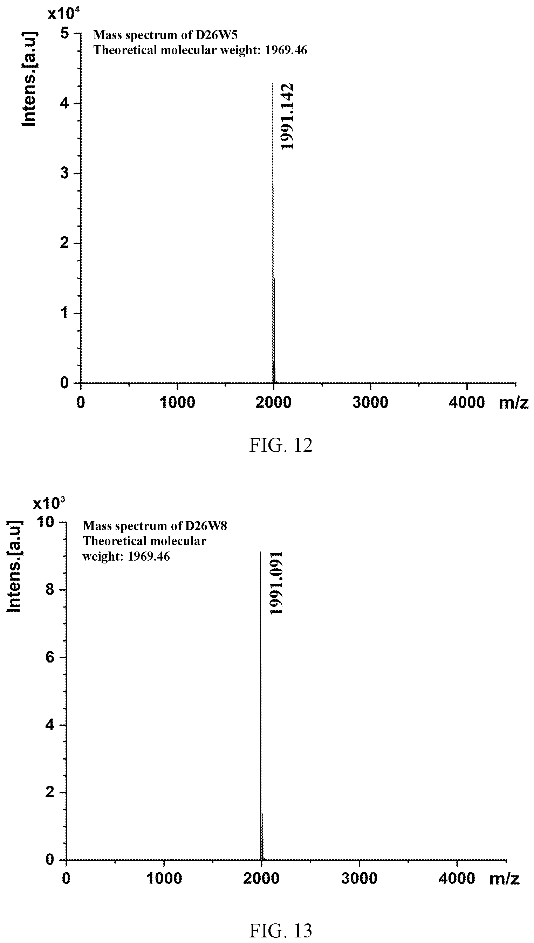

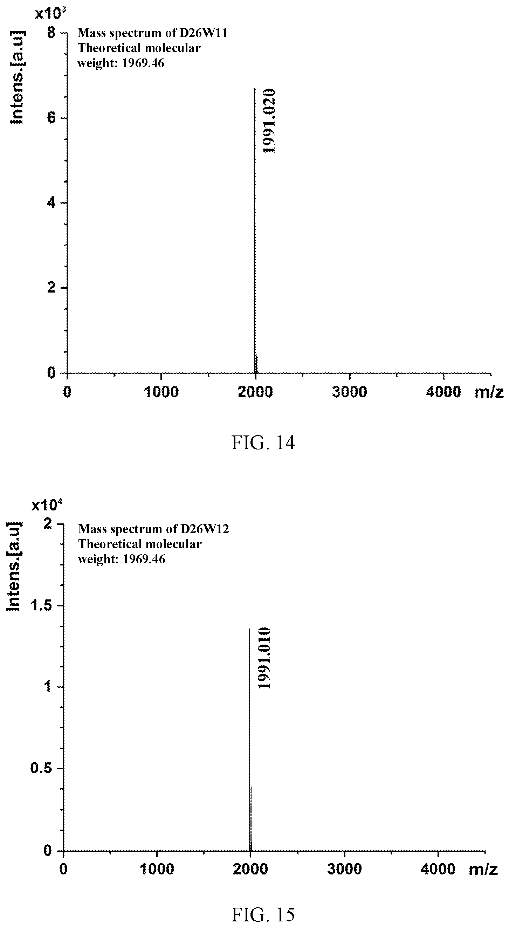

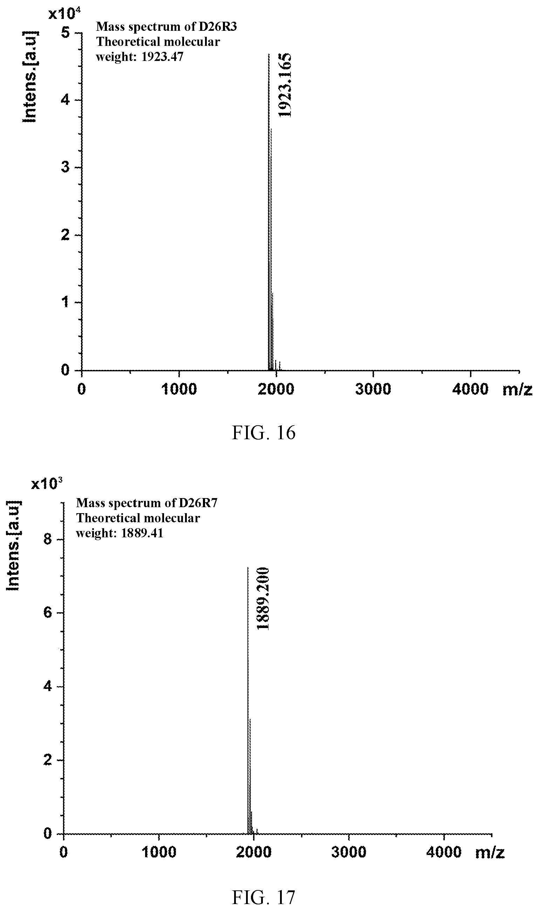

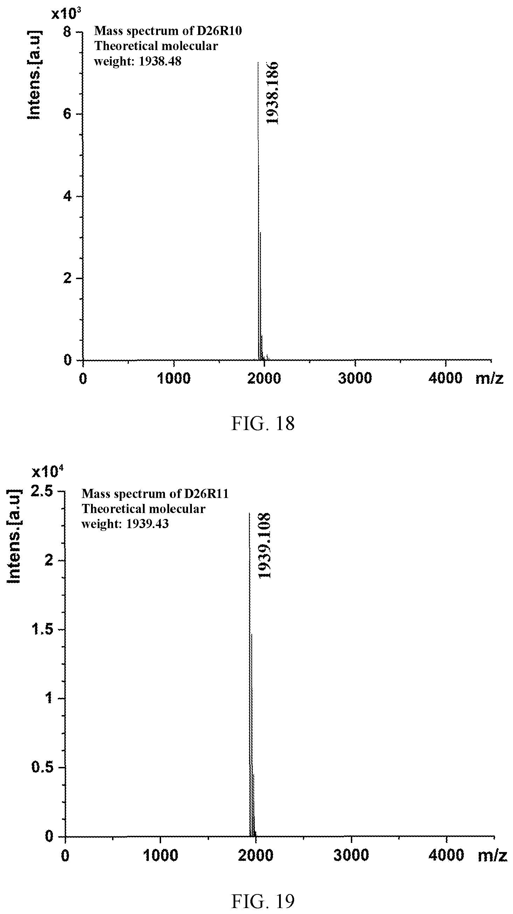

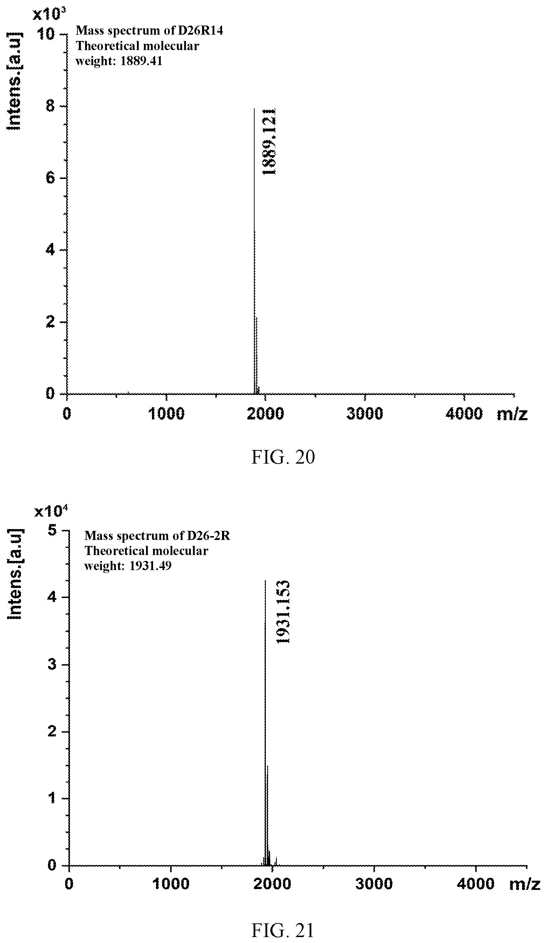

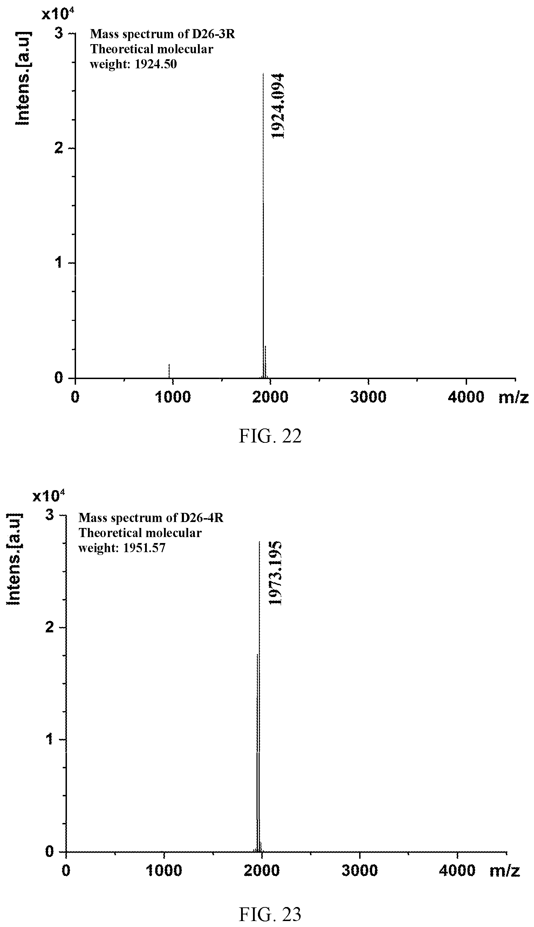

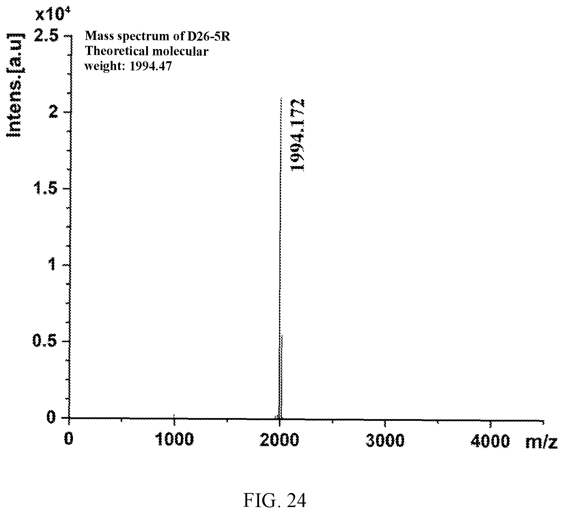

A small amount of the crude peptides were dissolved in water and acetonitrile and analyzed using an RP-C8 column (250 mm × 4.6 mm, 5 µm). The solvent system was water and acetonitrile (phase A: 0.1% CF₃COOH/H₂O; phase B: 1% CF₃COOH/90% CH₃CN/10% H₂O). The fraction corresponding to the main peak was collected, and the molecular weight was detected by MALDI-TOF mass spectrometer to confirm the correct polypeptide structure. The sequences and validation results of the generated polypeptides D26, D26W1, D26W4, D26W5, D26W8, D26W11, D26W12, D26R3, D26R7, D26R10, D26R11, D26R14, D26-2R, D26-3R, D26-4R, D26-5R, D1, F2, and F6 are shown in Table 1 and FIGs. 2-5 and 10-24. In Table 1, *¹⁻⁴ represent S5, ^{*1} and ^{*2} form an all-hydrocarbon chain linking bridge, and ^{*3} and ^{*4} form an all-hydrocarbon chain linking bridge.

**Table 1 Polypeptide sequences**

| name | **sequence** | **Theoretical Molecular Weight** | **Actual molecular weight** |
|---|---|---|---|
| D26 | Ac-G *¹ E A L *² Y L *³ N L L *⁴ Y W-NH₂ (SEQ ID NO:1) | 1896.4 | 1919.35 |
| D1 | Ac- *¹ W E A *² K *³ L W N *⁴ L Q Y W-NH₂ (SEQ ID NO:3) | 2078.62 | 2099.82 |
| F2 | Ac- G *¹ E A L *² Y L W N L L Q Y W-NH₂ (SEQ ID NO:8) | 1960.34 | 1982.83 |
| F6 | Ac-G W E A L K Y L *³ N L L *⁴ Y W-NH₂ (SEQ ID NO:9) | 1960.38 | 1982.96 |
| D26W1 | Ac-W *¹ EAL *² YL *³ NLL *⁴ YW-NH₂ (SEQ ID NO:10) | 2025.56 | 2047.56 |
| D26W4 | Ac-G *¹ EWL *² YL *³ NLL *⁴ YW-NH₂ (SEQ ID NO:11) | 2011.54 | 2033.02 |
| D26W5 | Ac-G *¹ EAW *² YL *³ NLL *⁴ YW-NH₂ (SEQ ID NO:12) | 1969.46 | 1991.14 |
| D26W8 | Ac-G *¹ EAL *² YW *³ NLL *⁴ YW-NH₂ (SEQ ID NO:13) | 1969.46 | 1991.09 |
| D26W11 | Ac-G *¹ EAL *² YL *³ NWL *⁴ YW-NH₂ (SEQ ID NO:14) | 1969.46 | 1991.02 |
| D26W12 | Ac-G *¹ EAL *² YL *³ NLW *⁴ YW-NH₂ (SEQ ID NO:15) | 1969.46 | 1991.01 |
| D26R3 | Ac-G *¹ RAL *² YL *³ NLL *⁴ YW-NH₂ (SEQ ID NO:16) | 1923.47 | 1923.16 |
| D26R7 | Ac-G *¹ EAL *² RL *³ NLL *⁴ YW-NH₂ (SEQ ID NO:17) | 1889.41 | 1889.20 |
| D26R10 | Ac-G *¹ EAL *² YL *³ RLL *⁴ YW-NH₂ (SEQ ID NO:18) | 1938.48 | 1938.19 |
| D26R11 | Ac-G *¹ EAL *² YL *³ NRL *⁴ YW-NH₂ (SEQ ID NO:19) | 1939.43 | 1939.11 |
| D26R14 | Ac-G *¹ EAL *² YL *³ NLL *⁴ RW-NH₂ (SEQ ID NO:20) | 1889.41 | 1889.12 |
| D26-2R | Ac-G *¹ EAL *² YL *³ RLL *⁴ RW-NH₂ (SEQ ID NO:21) | 1931.49 | 1931.15 |
| D26-3R | Ac-G *¹ EAL *² RL *³ RLL *⁴ RW-NH₂ (SEQ ID NO:22) | 1924.50 | 1924.09 |
| D26-4R | Ac-G *¹ RAL *² RL *³ RLL *⁴ RW-NH₂ (SEQ ID NO:23) | 1951.57 | 1951.20 |
| D26-5R | Ac-G *¹ RAL *² RL *³ RRL *⁴ RW-NH₂ (SEQ ID NO:24) | 1994.47 | 1994.17 |

In the purification of a polypeptide, the crude peptide was dissolved in water and acetonitrile, filtered through a 0.45 µm organic filter membrane, and then purified using LC-20A high-performance preparative liquid chromatography system. The chromatographic column was a C8 preparative column (250 mm × 30 mm, 5 µm), with binary gradient elution. The solvent system consisted of water and acetonitrile (phase A was 0.1% CF₃COOH/H₂O; phase B was 1% CF₃COOH/90% CH₃CN/10% H₂O); the dual wavelength channels were 210 nm and 254 nm; and the sample loading amount was 5 mL. After collecting the fraction from the target peak, the purity of the target peptide was analyzed by RP-HPLC high-performance liquid chromatography. The pure peptides were obtained by collection and lyophilization. The purification gradient conditions were as follows: 0-5 min 30-50% B; 5-10 min 50-70% B; 10-20 min 70-90% B; 20-30 min 90-100% B; 30-40 min 100% B, flow rate 15 mL/min, detection at 210 nm. Purity was determined using the RP-C8 column (250 mm × 4.6 mm, 5 µm) described above, with the mobile phase ratio remaining constant and the gradient conditions as follows: 0-5 min 10-50% B; 5-10 min 50-70% B; 10-15 min 70-90% B; 15-20 min 90-100% B; 25-30 min 100% B, flow rate 1 mL/min, detection at 210 nm. HPLC analysis showed that the purity was greater than 95.0%.

### Example 2. Anti-HIV-1 Activity Experiment

The infection inhibitory activity of laboratory-adapted HIV-1 X4 strain IIIB and HIV-1 R5 strain Bal (from the NIH AIDS Reagent Program, catalog numbers 398 and 510, respectively) was determined.

50 µL of PBS solutions containing 4.9, 19.5, 78.1, 312.5, 1250, and 5000 nM polypeptides D26, D26W1, D26W4, D26W5, D26W8, D26W11, D26W12, D26R3, D26R7, D26R10, D26R11, D26R14, D26-2R, D26-3R, D26-4R, D26-5R, D1, F2, F6, and F9170 were mixed with approximately 50 µL of live HIV-1 virus at approximately 100 times the 50% tissue culture infectious dose (TCID₅₀) and the mixture was incubated at 37 °C for 30 min. Then, 100 µL of MT-2 cells (for X4 virus) or CEMx174 5.25 M7 cells (for R5 virus) were added at 2 × 10⁵ cells/mL (MT-2 from NIH AIDS Reagent Program, catalog number 237; M7 cells from Sigma-Aldrich, catalog number 94022543) and incubated overnight at 37 °C. After replacing the supernatant with fresh RPMI-1640 medium containing 10% fetal bovine serum (FBS), the cells were cultured for another 3 days. 50 µL of culture supernatant was collected and mixed with an equal volume of 5% (v/v) Triton X-100. The p24 antigen in the final mixture was determined by ELISA. Simply put, these mixtures were added to a plate coated with 5 µg/mL of anti-HIV immunoglobulin (HIVIG) from the NIH AIDS Reagent Program. Anti-p24mAb183 (cultured from the NIH AIDS Reagent Program Anti-HIV-1 p24 Hybridoma cell line, catalog number: 1513), rabbit anti-mouse IgG-HRP (Dako) (Dako Denmark HRP-modified rabbit anti-mouse monoclonal antibody, catalog number P0260) and substrate 3,3,5,5-TMB (Sigma-Aldrich, catalog number 860336) were added sequentially. The absorbance at 450 nm (A450) was determined using a multimode microplate reader (Ultra 384, Tecan). The half inhibitory concentration was calculated using Calcusyn software (Biosoft), and plotted using GraphPad software.

The results are shown in Table 2, which demonstrate that D26, D26W1, D26W4, D26W5, D26W8, D26W11, D26W12, D26R3, D26R7, D26R10, D26R11, D26R14, D26-2R, D26-3R, D26-4R, D26-5R, D1, F2, and F6 can effectively inhibit the replication of laboratory-adapted HIV-1 IIIB and HIV-1 Bal strains, achieving or even exceeding the antiviral activity of F9170.

**Table 2 Virus inhibitory activity of the compounds**

| **Name** | **IC₅₀ (nM)** | |
|---|---|---|
| | **HIV-1 IIIB** | **HIV-1 BaL** |
| F9170 | 129.7 ± 88.5 | 77.0 ± 15.2 |
| D26 | 23.5 ± 13.7 | 200.8 ± 157.2 |
| D1 | 15.9 ± 12.0 | 83.4 ± 46.3 |
| F2 | 121.1 ± 42.2 | 269.3 ± 325.1 |
| F6 | 159.7 ± 34.0 | 13.4 ± 4.7 |
| D26W1 | 902.3 ± 75.7 | - |
| D26W4 | 989.5 ± 90.3 | - |
| D26W5 | 1416.0 ± 166.8 | - |
| D26W8 | 496.8 ± 102.6 | - |
| D26W11 | 1229.1 ± 600.1 | - |
| D26W12 | 773.9 ± 190.5 | - |
| D26R3 | 1232.1 ± 199.1 | - |
| D26R7 | 1436.1 ± 31.8 | - |
| D26R10 | 1055.3 ± 326.0 | - |
| D26R11 | 1038.5 ± 664.1 | - |
| D26R14 | 204.6 ± 36.7 | - |
| D26-2R | 423.1 ± 22.8 | - |
| D26-3R | 100.3 ± 13.4 | - |
| D26-4R | 417.2 ± 82.3 | - |
| D26-5R | 107.0 ± 17.9 | - |

### Example 3. Free HIV-1 Virus Particle Inactivation Experiment

50 µL of 200-fold TCID50 HIV-1 X4 strain IIIB and HIV-1 R5 strain Bal were mixed with 50 µL of PBS solutions containing 4.9, 19.5, 78.1, 312.5, 1250 and 5000 nM polypeptides D26, D1, F2, F6 and F9170, and incubated at 4 °C for 1 h. 15% PEG-6000, 25 µL/well was added to a final concentration of 3%, and incubated at 4 °C for 1 h. The polypeptide solution was centrifuged at 4 °C and 13000 rpm/min for 30 min, and the compounds in the supernatant were discarded. The virus was resuspended in 3% PEG-6000 containing 10 mg/mL BSA at 4 °C and 13000 rpm/min, and the residual compounds in the supernatant were discarded. The steps above were repeated once. The virus was resuspended in 100 µL of serum-free medium per well and 3 × 10⁴ per well of the corresponding target cells diluted with complete medium were added. The experiment included both a cell control (comprising only cells, without virus or drug) and a virus control (comprising no drug, but with virus and corresponding target cells). Both were incubated in a 37 °C, 5% CO₂ incubator. On day 4, 50 µL of supernatant per well was collected and 50 µL of 5% Triton-100 per well was added to lyse the virus. The inactivation activity of the compound against the virus was determined by detecting the p24 antigen in the supernatant using a double-antibody sandwich ELISA.

The results are shown in Table 3, which demonstrate that D26, D1, F2, and F6 can highly effectively inactivate laboratory-adapted strains HIV-1 IIIB and HIV-1 Bal, with activity comparable to that of F9170.

**Table 3 Virus inactivation activity of the compounds**

| **Name** | **EC₅₀ (nM)** | |
|---|---|---|
| | **HIV-1 IIIB** | **HIV-1 BaL** |
| F9170 | 26.1 ± 9.9 | 25.3 ± 20.1 |
| D26 | 8.2 ± 3.1 | 36.7 ± 15.8 |
| D1 | 58.1 ± 14.3 | 4.3 ± 5.3 |
| F2 | 121.1 ± 42.2 | 269.3 ± 325.1 |
| F6 | 95.5 ± 24.157 | 22.0 ± 11.0 |

### Example 4. Determination of Half-life in Rats

Eighteen SD rats (female : male = 1 : 1) (purchased from Vital River Laboratories) were randomly divided into three groups and injected intravenously with 1 mg/kg of D26, D1, F2, F6, D26W1, D26W4, D26W5, D26W8, D26W11, D26W12, or F9170, respectively. 100 µL of blood sample was collected from the orbital cavity at 2 min, 5 min, 15 min, 30 min, 1 h, 2 h, 4 h, 6 h, 8 h, 12 h, 1 d, 2 d, 3 d, 4 d, 5 d, 6 d, and 8 d following injection. The supernatant was collected by centrifuging the blood sample at 3000 rpm/min for 20 min at 4 °C and stored at -20 °C, and the sample concentration was determined by LC-MS (Shimadzu, Japan). The half-life of the polypeptide in mice was calculated using the non-compartmental model module in the software Phoenix WinNonlin (version 8.2, Certara, U.S.A.).

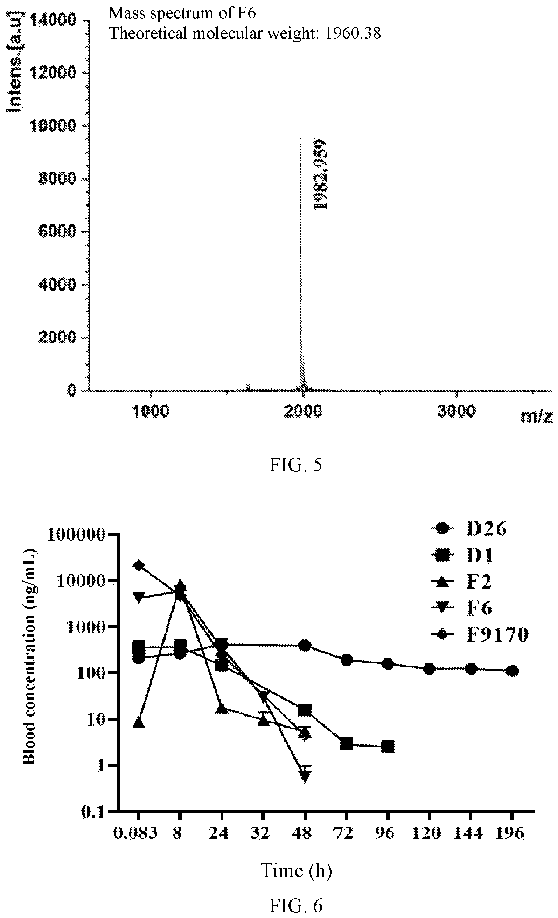

The results are shown in Table 4 and FIG. 6, which show that the half-life of F9170 is only 3.89 h, while the half-life of D26 in rats is significantly prolonged to 86.6 h. The half-life of F2 and F6 modified by the single-bridged stapling strategy is basically the same as that of F9170. The polypeptides D1, D26W1, D26W4, D26W5, D26W8, D26W11, D26W12, D26R3, D26R7, D26R10, D26R11, D26R14, D26-2R, and D26-3R, which are modified using the same double-bridged stapling strategy as D26, have a half-life that is 2-26 times longer than that of F9170. This demonstrates the unique advantages of double-bridged stapled peptides such as D26, D1, D26W1, D26W4, D26W5, D26W8, D26W11, D26W12, D26R3, D26R7, D26R10, D26R11, D26R14, D26-2R, and D26-3R.

**Table 4 In vivo half-life of the compounds**

| **Name** | **t_{1/2}(h)** |
|---|---|
| F9170 | 3.89 ± 0.27 |
| D26 | 86.61 ± 7.21 |
| D1 | 8.7 ± 0.44 |
| F2 | 4.12 ± 0.35 |
| F6 | 3.60 ± 0.54 |
| D26W1 | 9.55 ± 1.37 |
| D26W4 | 42.03 ± 3.15 |
| D26W5 | 48.31 ± 9.38 |
| D26W8 | 52.15 ± 7.69 |
| D26W11 | 23.23 ± 3.71 |
| D26W12 | 7.66 ± 0.99 |
| D26R3 | 56.27 ± 5.85 |
| D26R7 | 43.78 ± 5.07 |
| D26R10 | 59.60 ± 3.12 |
| D26R11 | 19.90 ± 0.61 |
| D26R14 | 72.56 ± 12.86 |
| D26-2R | 100.48 ± 62.15 |
| D26-3R | 57.45 ± 6.19 |

### Example 5. Rat Tissue Exposure Experiment

Thirty SD rats were randomly divided into two groups and injected intravenously with 1 mg/kg D26 or F9170 according to the mice's body weight. At 5 min, 2 h, 4 h, 6 d, and 12 d after injection, tissues and organs (brain, testis, spleen, small intestine and cervical lymph nodes) of the three mice were collected and stored at -20 °C. After the tissue homogenate was prepared, the concentration of D26 or F9170 in the tissue was determined by LC-MS, and the AUC (Area Under Curve) of D26 and F9170 in each tissue was calculated. AUC (Area Under Curve) is calculated by the non-compartmental model module in the software Phoenix WinNonlin (version 8.2, Certara, U.S.A.).

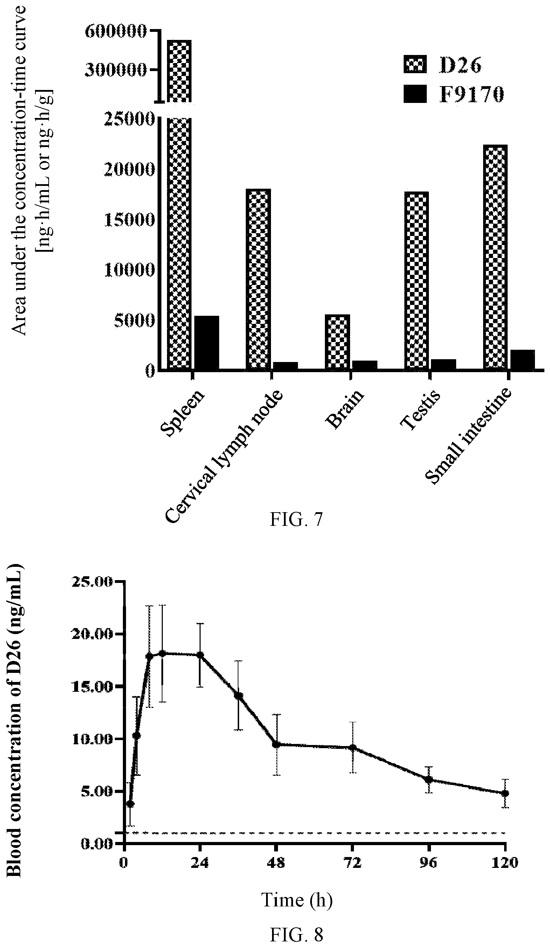

The results, as shown in Table 5 and FIG. 7, indicate that the AUC of D26 in the HIV anatomical reservoir, comprising the brain, spleen, small intestine, cervical lymph nodes, and testes, is much higher than that of F9170, suggesting that D26 can be better distributed in the anatomical reservoir and kill HIV within them.

**Table 5 AUC of compounds in various tissues**

| **Tissue** | **AUC _{(0-288 hours)} hours * ng/(mL or g)** | |
|---|---|---|
| | **D26** | **F9170** |
| Brain | 5562.361 | 1002.761 |
| Testis | 17772.679 | 1138.907 |
| Spleen | 531298.12 | 5429.07 |
| Small intestine | 22413.262 | 2074.9604 |
| Cervical lymph node | 18053.554 | 842.45886 |

### Example 6. Oral Absorption Experiment

Twelve SD rats were randomly divided into two groups, and were administered 50 mg/kg D26 or F9170 by gavage, respectively. 100 µL of orbital blood samples were collected at 2 h, 4 h, 6 h, 8 h, 12 h, 1 d, 2 d, 3 d, 4 d, 5 d, 6 d, and 8 d after gavage administration. The sample was centrifuged at 4 °C, 3000 rpm/min for 20 min, and the supernatant was collected and stored at -80 °C until LC-MS was used to determine the sample concentration.

The results are shown in FIG. 8, which shows that no signal was detected in the blood samples of group F9170, while a significant signal was detected in group D26 and lasted for at least 5 days. This indicates that D26 can be absorbed into the bloodstream through the gastrointestinal tract, suggesting it has oral administration potential.

### Example 7. Safety Profile

Eighteen 6-8 week old BALB/c mice (purchased from SPAF (Beijing) Biotechnology Co., Ltd.) were randomly divided into three groups. The mice were intravenously injected with 20 mg/kg, 100 mg/kg and an equal volume of PBS, respectively. The weight changes of the mice were monitored at different time points after injection, and blood samples were collected. The serum transaminase and serum creatinine levels were determined using a serum transaminase and serum creatinine assay kit (NJJCBIO).

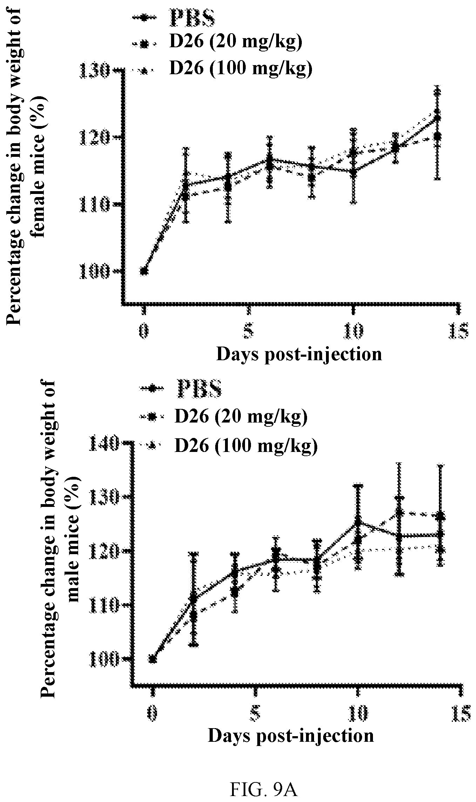

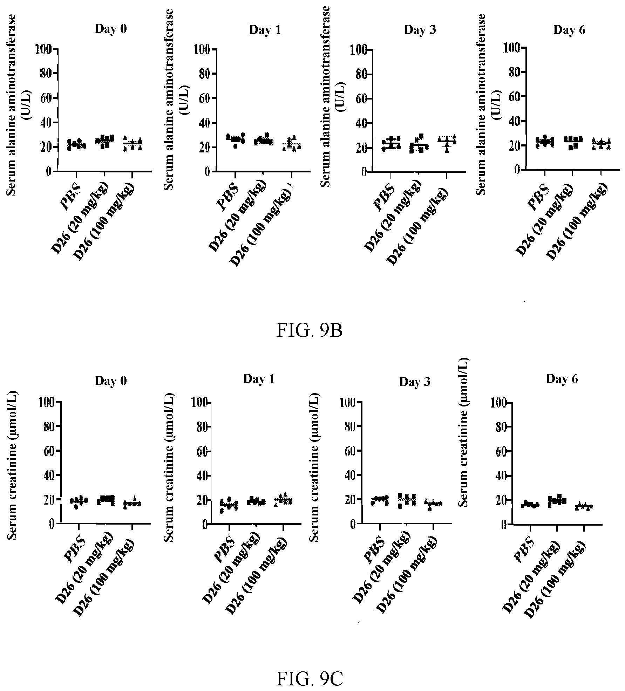

The results, as shown in FIGs. 9A-C, indicate that at doses of D26 of 20 mg/kg and 100 mg/kg, there was no significant difference in body weight change between the mice and the PBS group, nor were there significant differences in serum transaminase and serum creatinine levels between the mice and the PBS group. This demonstrates the good in vivo safety of D26.

## Claims

1. A polypeptide, or a stereoisomer, derivative, pharmaceutically acceptable salt, mixture, or functionally equivalent variant thereof, wherein the polypeptide comprises an amino acid sequence represented by any one of the following general formulas (I)-(IV), or an amino acid sequence represented by any one of the following general formulas (I)-(IV) having 1-5 conservative substitutions:
X₁-X₂-X₃-X₄-X₅-X₆-X₇-X₈-X₉-X₁₀-X₁₁-X₁₂-X₁₃-X₁₄-W (I),
X₂-W-E-A-X₆-K-X₉-L-W-N-X₁₃-L-Q-Y-W (II),
G-X₂-E-A-L-X₆-Y-L-W-N-L-L-Q-Y-W (III),
G-W-E-A-L-K-Y-L-X₉-N-L-L-X₁₃-Y-W (IV),
wherein X₁ represents any amino acid, preferably G or W, or an amino acid residue having similar properties thereto;
X₂, X₆, X₉, and X₁₃ are independently non-natural amino acids, wherein (X₂) and (X₆), and (X₉) and (X₁₃) are bonded to each other via a covalent bond linkage, respectively;
X₃ represents any amino acid, preferably E or R, or an amino acid residue having similar properties thereto;
X₄ represents any amino acid, preferably A or W, or an amino acid residue having similar properties thereto;
X₅ represents any amino acid, preferably L or W, or an amino acid residue having similar properties thereto;
X₇ represents any amino acid, preferably Y or R, or an amino acid residue having similar properties thereto;
X₈ represents any amino acid, preferably L or W, or an amino acid residue having similar properties thereto;
X₁₀ represents any amino acid, preferably N or R, or an amino acid residue having similar properties thereto;
X₁₁ represents any amino acid, preferably L, W, or R, or an amino acid residue having similar properties thereto;
X₁₂ represents any amino acid, preferably L or W, or an amino acid residue having similar properties thereto;
X₁₄ represents any amino acid, preferably Y or R, or an amino acid residue having similar properties thereto; and
the symbol "-" represents a peptide bond between amino acid residues.

2. The polypeptide, or the stereoisomer, derivative, pharmaceutically acceptable salt, mixture, or functionally equivalent variant of the polypeptide according to claim 1, wherein the polypeptide comprises an amino acid sequence represented by the following general formula (V), or an amino acid sequence represented by the following general formula (V) having 1-5 conservative substitutions:
G-X₂-X₃-A-L-X₆-X₇-L-X₉-X₁₀-X₁₁-L-X₁₃-X₁₄-W (V),
wherein X₂, X₆, X₉, and X₁₃ are independently non-natural amino acids, wherein (X₂) and (X₆), and (X₉) and (X₁₃) are bonded to each other via a covalent bond linkage, respectively;
X₃ represents any amino acid, preferably E or R, or an amino acid residue having similar properties thereto;
X₇ represents any amino acid, preferably Y or R, or an amino acid residue having similar properties thereto;
X₁₀ represents any amino acid, preferably N or R, or an amino acid residue having similar properties thereto;
X₁₁ represents any amino acid, preferably L or R, or an amino acid residue having similar properties thereto;
X₁₄ represents any amino acid, preferably Y or R, or an amino acid residue having similar properties thereto; and
the symbol "-" represents a peptide bond between amino acid residues.

3. The polypeptide, or the stereoisomer, derivative, pharmaceutically acceptable salt, mixture, or functionally equivalent variant of the polypeptide according to claim 1 or 2, wherein
preferably, the non-natural amino acids are independently selected from amino acids with olefin side chains, such as (*S*)-2-(4'-pentenyl)alanine (i.e. S5), (*R*)-2-(4'-pentenyl)alanine (i.e. R5), (*S*)-2-(7'-octenyl)alanine (i.e. S8), (*R*)-2-(7'-octenyl)alanine (i.e. R8), and 2-amino-2-(4'-pentenyl)-6-heptenoic acid (i.e. B5); more preferably, the non-natural amino acid is (*S*)-2-(4'-pentenyl)alanine (i.e. S5); and
preferably, the covalent bond linkage comprises a disulfide bond, a hydrocarbon chain, an intramolecular amide bond, a thioether bond, or a covalent bond linkage formed by an azide-alkyne cycloaddition reaction; more preferably, the covalent bond linkage is a covalent bond linkage formed by olefin metathesis, such as an all-hydrocarbon chain linking bridge.

4. The polypeptide, or the stereoisomer, derivative, pharmaceutically acceptable salt, mixture, or functionally equivalent variant of the polypeptide according to any one of claims 1 to 3, wherein the polypeptide comprises the amino acid sequence of any one of (a)-(t):
(a) an amino acid sequence having at least 65%, at least 70%, at least 75%, at least 80%, at least 85%, or at least 90% sequence identity to an amino acid sequence set forth in SEQ ID NO: 1, or an amino acid sequence having 1-5 conservative substitutions relative to the amino acid sequence set forth in SEQ ID NO: 1, preferably the amino acid sequence set forth in SEQ ID NO: 1;
(b) an amino acid sequence having at least 65%, at least 70%, at least 75%, at least 80%, at least 85%, or at least 90% sequence identity to an amino acid sequence set forth in SEQ ID NO: 2, or an amino acid sequence having 1-5 conservative substitutions relative to the amino acid sequence set forth in SEQ ID NO: 2, preferably the amino acid sequence A(S5)EAL(S5)YL(S5)NLL(S5)YW set forth in SEQ ID NO: 2, wherein the first and second S5 residues are covalently linked, and the third and fourth S5 residues are covalently linked;
(c) an amino acid sequence having at least 65%, at least 70%, at least 75%, at least 80%, at least 85%, or at least 90% sequence identity to an amino acid sequence set forth in SEQ ID NO: 3, or an amino acid sequence having 1-5 conservative substitutions relative to the amino acid sequence set forth in SEQ ID NO: 3, preferably the amino acid sequence set forth in SEQ ID NO: 3;
(d) an amino acid sequence having at least 65%, at least 70%, at least 75%, at least 80%, at least 85%, or at least 90% sequence identity to an amino acid sequence set forth in SEQ ID NO: 10, or an amino acid sequence having 1-5 conservative substitutions relative to the amino acid sequence set forth in SEQ ID NO: 10, preferably the amino acid sequence set forth in SEQ ID NO: 10;
(e) an amino acid sequence having at least 65%, at least 70%, at least 75%, at least 80%, at least 85%, or at least 90% sequence identity to an amino acid sequence set forth in SEQ ID NO: 11, or an amino acid sequence having 1-5 conservative substitutions relative to the amino acid sequence set forth in SEQ ID NO: 11, preferably the amino acid sequence set forth in SEQ ID NO: 11;
(f) an amino acid sequence having at least 65%, at least 70%, at least 75%, at least 80%, at least 85%, or at least 90% sequence identity to an amino acid sequence set forth in SEQ ID NO: 12, or an amino acid sequence having 1-5 conservative substitutions relative to the amino acid sequence set forth in SEQ ID NO: 12, preferably the amino acid sequence set forth in SEQ ID NO: 12;
(g) an amino acid sequence having at least 65%, at least 70%, at least 75%, at least 80%, at least 85%, or at least 90% sequence identity to an amino acid sequence set forth in SEQ ID NO: 13, or an amino acid sequence having 1-5 conservative substitutions relative to the amino acid sequence set forth in SEQ ID NO: 13, preferably the amino acid sequence set forth in SEQ ID NO: 13;
(h) an amino acid sequence having at least 65%, at least 70%, at least 75%, at least 80%, at least 85%, or at least 90% sequence identity to an amino acid sequence set forth in SEQ ID NO: 14, or an amino acid sequence having 1-5 conservative substitutions relative to the amino acid sequence set forth in SEQ ID NO: 14, preferably the amino acid sequence set forth in SEQ ID NO: 14;
(i) an amino acid sequence having at least 65%, at least 70%, at least 75%, at least 80%, at least 85%, or at least 90% sequence identity to an amino acid sequence set forth in SEQ ID NO: 15, or an amino acid sequence having 1-5 conservative substitutions relative to the amino acid sequence set forth in SEQ ID NO: 15, preferably the amino acid sequence set forth in SEQ ID NO: 15;
(j) an amino acid sequence having at least 65%, at least 70%, at least 75%, at least 80%, at least 85%, or at least 90% sequence identity to an amino acid sequence set forth in SEQ ID NO: 16, or an amino acid sequence having 1-5 conservative substitutions relative to the amino acid sequence set forth in SEQ ID NO: 16, preferably the amino acid sequence set forth in SEQ ID NO: 16;
(k) an amino acid sequence having at least 65%, at least 70%, at least 75%, at least 80%, at least 85%, or at least 90% sequence identity to an amino acid sequence set forth in SEQ ID NO: 17, or an amino acid sequence having 1-5 conservative substitutions relative to the amino acid sequence set forth in SEQ ID NO: 17, preferably the amino acid sequence set forth in SEQ ID NO: 17;
(l) an amino acid sequence having at least 65%, at least 70%, at least 75%, at least 80%, at least 85%, or at least 90% sequence identity to an amino acid sequence set forth in SEQ ID NO: 18, or an amino acid sequence having 1-5 conservative substitutions relative to the amino acid sequence set forth in SEQ ID NO: 18, preferably the amino acid sequence set forth in SEQ ID NO: 18;
(m) an amino acid sequence having at least 65%, at least 70%, at least 75%, at least 80%, at least 85%, or at least 90% sequence identity to an amino acid sequence set forth in SEQ ID NO: 19, or an amino acid sequence having 1-5 conservative substitutions relative to the amino acid sequence set forth in SEQ ID NO: 19, preferably the amino acid sequence set forth in SEQ ID NO: 19;
(n) an amino acid sequence having at least 65%, at least 70%, at least 75%, at least 80%, at least 85%, or at least 90% sequence identity to an amino acid sequence set forth in SEQ ID NO: 20, or an amino acid sequence having 1-5 conservative substitutions relative to the amino acid sequence set forth in SEQ ID NO: 20, preferably the amino acid sequence set forth in SEQ ID NO: 20;
(o) an amino acid sequence having at least 65%, at least 70%, at least 75%, at least 80%, at least 85%, or at least 90% sequence identity to an amino acid sequence set forth in SEQ ID NO: 21, or an amino acid sequence having 1-5 conservative substitutions relative to the amino acid sequence set forth in SEQ ID NO: 21, preferably the amino acid sequence set forth in SEQ ID NO: 21;
(p) an amino acid sequence having at least 65%, at least 70%, at least 75%, at least 80%, at least 85%, or at least 90% sequence identity to an amino acid sequence set forth in SEQ ID NO: 22, or an amino acid sequence having 1-5 conservative substitutions relative to the amino acid sequence set forth in SEQ ID NO: 22, preferably the amino acid sequence set forth in SEQ ID NO: 22;
(q) an amino acid sequence having at least 65%, at least 70%, at least 75%, at least 80%, at least 85%, or at least 90% sequence identity to an amino acid sequence set forth in SEQ ID NO: 23, or an amino acid sequence having 1-5 conservative substitutions relative to the amino acid sequence set forth in SEQ ID NO: 23, preferably the amino acid sequence set forth in SEQ ID NO: 23;
(r) an amino acid sequence having at least 65%, at least 70%, at least 75%, at least 80%, at least 85%, or at least 90% sequence identity to an amino acid sequence set forth in SEQ ID NO: 24, or an amino acid sequence having 1-5 conservative substitutions relative to the amino acid sequence set forth in SEQ ID NO: 24, preferably the amino acid sequence set forth in SEQ ID NO: 24;
(s) an amino acid sequence having at least 65%, at least 70%, at least 75%, at least 80%, at least 85%, or at least 90% sequence identity to an amino acid sequence set forth in SEQ ID NO: 8, or an amino acid sequence having 1-5 conservative substitutions relative to the amino acid sequence set forth in SEQ ID NO: 8, preferably the amino acid sequence set forth in SEQ ID NO: 8; and
(t) an amino acid sequence having at least 65%, at least 70%, at least 75%, at least 80%, at least 85%, or at least 90% sequence identity to an amino acid sequence set forth in SEQ ID NO: 9, or an amino acid sequence having 1-5 conservative substitutions relative to the amino acid sequence set forth in SEQ ID NO: 9, preferably the amino acid sequence set forth in SEQ ID NO: 9.

5. The polypeptide, or the stereoisomer, derivative, pharmaceutically acceptable salt, mixture, or functionally equivalent variant of the polypeptide according to any one of claims 1 to 4, has one or more of the following advantages: simple synthesis, low synthesis cost, oral administration capability or oral administration potential, high antiviral activity for inhibiting HIV replication, high activity for lysing HIV viral particles, high activity for inactivating free HIV particles, long-acting effect, effective entry into an HIV anatomical reservoir, and good biosafety; preferably, compared with F9170, the polypeptide, or the stereoisomer, derivative, pharmaceutically acceptable salt, mixture, or functionally equivalent variant of the polypeptide, has improved half-life, oral administration capability or oral administration potential, and/or effective entry into an HIV anatomical reservoir.

6. The polypeptide, or the stereoisomer, derivative, pharmaceutically acceptable salt, mixture, or functionally equivalent variant of the polypeptide according to any one of claims 1 to 5, wherein the derivative is selected from a derivative obtained by maleimide modification of the polypeptide, a derivative obtained by attaching an amino-terminal protecting group to the N-terminus and/or a carboxyl-terminal protecting group to the C-terminus, a derivative obtained by protein or polyethylene glycol modification of the polypeptide, a derivative obtained by attaching an oligopeptide, a lipophilic group, or cholesterol to the N-terminus and/or the C-terminus, or a derivative obtained by substituting one or more amino acid residues with D-amino acids, artificially modified amino acids, or naturally occurring rare amino acids.

7. The polypeptide, or the stereoisomer, derivative, pharmaceutically acceptable salt, mixture, or functionally equivalent variant of the polypeptide according to claim 6, wherein the amino-terminal protecting group is selected from: H, a polyethylene glycol-derived polymer, an unsubstituted or substituted acyclic aliphatic group, an unsubstituted or substituted alicyclic group, an unsubstituted or substituted heterocyclic group, an unsubstituted or substituted heteroarylalkyl group, an unsubstituted or substituted aryl group, an unsubstituted or substituted aralkyl group, and R₅-CO-, wherein R₅ is selected from: H, an unsubstituted or substituted acyclic aliphatic group, an unsubstituted or substituted alicyclic group, an unsubstituted or substituted aryl group, an unsubstituted or substituted aralkyl group, an unsubstituted or substituted heterocyclic group, and an unsubstituted or substituted heteroarylalkyl group; preferably, the amino-terminal protecting group is selected from: H, acetyl (Ac), lauroyl, myristoyl, and palmitoyl; and
the carboxyl-terminal protecting group is selected from: -NR₃R₄, -OR₃, and -SR₃, wherein R₃ and R₄ are independently selected from: H, a polyethylene glycol-derived polymer, an unsubstituted or substituted acyclic aliphatic group, an unsubstituted or substituted alicyclic group, an unsubstituted or substituted heterocyclic group, an unsubstituted or substituted heteroarylalkyl group, an unsubstituted or substituted aryl group, and an unsubstituted or substituted aralkyl group; preferably, the carboxyl-terminal protecting group is selected from: -NR₃R₄ and -OR₃, wherein R₃ and R₄ are independently selected from H, methyl, ethyl, hexyl, dodecyl, and hexadecyl.

8. A pharmaceutical composition, drug conjugate, drug complex, or fusion protein, wherein the pharmaceutical composition comprises the polypeptide, or the stereoisomer, derivative, pharmaceutically acceptable salt, mixture, or functionally equivalent variant of the polypeptide according to any one of claims 1 to 7, and a pharmaceutically acceptable carrier or excipient, and optionally further comprises one or more additional HIV therapeutic agents or HIV latency-reversing agents; the drug conjugate or drug complex comprises the polypeptide, or the stereoisomer, derivative, pharmaceutically acceptable salt, mixture, or functionally equivalent variant of the polypeptide according to any one of claims 1 to 7, optionally comprises a carrier protein, such as serum albumin, immunoglobulin, ferritin, transferrin, α-2-macroglobulin, thyroxine-binding protein, or steroid-binding protein, and optionally comprises one or more additional HIV therapeutic agents or HIV latency-reversing agents; and the fusion protein comprises the polypeptide, or the stereoisomer, derivative, pharmaceutically acceptable salt, mixture, or functionally equivalent variant of the polypeptide according to any one of claims 1 to 7, and optionally comprises one or more additional proteins for treating or preventing HIV infection, such as an antibody against an intracellular domain of an HIV envelope protein, preferably an antibody against LLP1-GQ.

9. A method for treating or preventing diseases caused by HIV infection, comprising administering to a subject in need thereof a therapeutically effective amount of the polypeptide, or the stereoisomer, derivative, pharmaceutically acceptable salt, mixture, or functionally equivalent variant of the polypeptide according to any one of claims 1 to 7, or the pharmaceutical composition, drug conjugate, drug complex, or fusion protein according to claim 8.

10. The method according to claim 9, wherein the polypeptide, or the stereoisomer, derivative, pharmaceutically acceptable salt, mixture, or functionally equivalent variant of the polypeptide, the pharmaceutical composition, the drug conjugate, the drug complex, or the fusion protein is administered in combination with one or more additional HIV therapeutic agents, wherein the one or more additional HIV therapeutic agents comprise reverse transcriptase inhibitors, protease inhibitors, entry inhibitors, integrase inhibitors, and maturation inhibitors.

11. An in vitro method for any one or more of the following (a)-(e):
(a) inhibiting HIV infection and/or replication;
(b) inactivating free HIV viral particles;
(c) preparing a formulation having in vitro HIV infection-inhibitory activity;
(d) eliminating viruses in an HIV reservoir or reducing the number of viruses in the HIV reservoir; and
(e) lysing HIV viral particles, wherein
the method comprises using the polypeptide, or the stereoisomer, derivative, pharmaceutically acceptable salt, mixture, or functionally equivalent variant of the polypeptide according to any one of claims 1 to 7.

12. A method for producing the polypeptide, or the stereoisomer, derivative, pharmaceutically acceptable salt, mixture, or functionally equivalent variant of the polypeptide according to any one of claims 1 to 7, or the fusion protein according to claim 8, comprising chemically synthesizing the polypeptide, or the stereoisomer, derivative, pharmaceutically acceptable salt, mixture, or functionally equivalent variant of the polypeptide, or the fusion protein.
